# FASCICULE DE BREVET EUROPEEN

(11) **EP 2 414 839 B1**
(45) Date de publication et mention de la délivrance du brevet: **25.06.2014**
(21) Numéro de dépôt: 10716574.8
(22) Date de dépôt: 25.03.2010
(51) Int. Cl.: G01N 33/569, G01N 33/576

(54) **SUPPORT SOLIDE DE DÉTECTION DU VHC**
FESTER TRÄGER ZUM NACHWEIS VON HCV
SOLID SUPPORT FOR THE DETECTION OF HCV

(30) Priorité: 30.03.2009 FR 0951949
(43) Date de publication de la demande: 08.02.2012
(73) Titulaire: Biomérieux, 69280 Marcy L'etoile (FR)
(72) Inventeur: BERLAND, Jean-Luc, 38300 Succieu (FR); PARANHOS-BACCALA, Glaucia, 69003 Lyon (FR)
(74) Mandataire: Bitaud, Valérie Marie-Odile
(86) Numéro de dépôt international: PCT/FR2010/050538
(87) Numéro de publication internationale: WO 2010/112733

(56) Documents cités:
- EP-A2- 1 251 353
- WO-A1-2004/070387
- WO-A2-01/38360
- WO-A2-01/96875
- WO-A2-03/002749
- WO-A2-03/095968
- WO-A2-2008/027942
- MORADPOUR DARIUS ET AL: "Replication of hepatitis C virus." NATURE REVIEWS. MICROBIOLOGY JUN 2007, vol. 5, no. 6, juin 2007 (2007-06), pages 453-463, XP002555991 ISSN: 1740-1534
- SILLANPÄÄ MAARIT ET AL: "Hepatitis C virus core, NS3, NS4B and NS5A are the major immunogenic proteins in humoral immunity in chronic HCV infection." VIROLOGY JOURNAL 2009 LNKD- PUBMED:19549310, vol. 6, 2009, page 84, XP002585956 ISSN: 1743-422X

### SEQUENCE LISTING

<110> bioMérieux
<120> Support solide de détection du VHC
<130> HCV Combo
<150> FR09 51949
   <151> 2009-03-30
<160> 16
<170> Patent In version 3.3
<210> 1
   <211> 302
   <212> PRT
   <213> Artificial sequence
<220>
   <223> E2 cloné du VHC
<400> 1
   Met Arg Gly Ser His His His Thr His Val Thr Gly Gly Arg Val Ala
   1               5                   10                  15
   Ser Ser Thr Gln Ser Leu Val Ser Trp Leu Ser Gln Gly Pro Ser Gln
               20                  25                  30
   Lys Ile Gln Leu Val Asn Thr Asn Gly Ser Trp His Ile Asn Arg Thr
           35                  40                  45
   Ala Leu Asn Cys Asn Asp Ser Leu Gln Thr Gly Phe Ile Ala Ala Leu
       50                  55                  60
   Phe Tyr Ala His Arg Phe Asn Ala Ser Gly Cys Pro Glu Arg Met Ala
   65                  70                  75                  80
   Ser Cys Arg Pro Ile Asp Lys Phe Ala Gln Gly Trp Gly Pro Ile Thr
                   85                  90                  95
   His Val Val Pro Asn Ile Ser Asp Gln Arg Pro Tyr Cys Trp His Tyr
               100                 105                 110
   Ala Pro Gln Pro Cys Gly Ile Val Pro Ala Ser Gln Val Cys Gly Pro
           115                 120                 125
   Val Tyr Cys Phe Thr Pro Ser Pro Val Val Val Gly Thr Thr Asp Arg
       130                 135                 140
   Ser Gly Val Pro Thr Tyr Ser Trp Gly Glu Asn Glu Thr Asp Val Leu
   145                 150                 155                 160
   Leu Leu Asn Asn Thr Arg Pro Pro Gln Gly Asn Trp Phe Gly Cys Thr
                   165                 170                 175
   Trp Met Asn Ser Thr Gly Phe Thr Lys Thr Cys Gly Gly Pro Pro Cys
   180 185 190
   Asn Ile Gly Gly Val Gly Asn Asn Thr Leu Ile Cys Pro Thr Asp Cys
   195 200 205
   Phe Arg Lys His Pro Glu Ala Thr Tyr Thr Lys Cys Gly Ser Gly Pro
   210 215 220
   Trp Leu Thr Pro Arg Cys Leu Val Asp Tyr Pro Tyr Arg Leu Trp His
   225 230 235 240
   Tyr Pro Cys Thr Ile Asn Phe Thr Ile Phe Lys Val Arg Met Tyr Val
   245 250 255
   Gly Gly Val Glu His Arg Leu Asn Ala Ala Cys Asn Trp Thr Arg Gly
   260 265 270
   Glu Arg Cys Asp Leu Glu Asp Arg Asp Arg Ser Glu Leu Ser Pro Leu
   275 280 285
   Leu Leu Ser Thr Thr Glu Trp Gln His His His His His His
   290 295 300
<210> 2
   <211> 288
   <212> PRT
   <213> Hepatitis C virus
<400> 2
   His Val Thr Gly Gly Arg Val Ala Ser Ser Thr Gln Ser Leu Val Ser
   1               5                   10                  15
   Trp Leu Ser Gln Gly Pro Ser Gln Lys Ile Gln Leu Val Asn Thr Asn
               20                  25                  30
   Gly Ser Trp His Ile Asn Arg Thr Ala Leu Asn Cys Asn Asp Ser Leu
           35                  40                  45
   Gln Thr Gly Phe Ile Ala Ala Leu Phe Tyr Ala His Arg Phe Asn Ala
       50                  55                  60
   Ser Gly Cys Pro Glu Arg Met Ala Ser Cys Arg Pro Ile Asp Lys Phe
   65                  70                  75                  80
   Ala Gln Gly Trp Gly Pro Ile Thr His Val Val Pro Asn Ile Ser Asp
                   85                  90                  95
   Gln Arg Pro Tyr Cys Trp His Tyr Ala Pro Gln Pro Cys Gly Ile Val
               100                 105                 110
   Pro Ala Ser Gln Val Cys Gly Pro Val Tyr Cys Phe Thr Pro Ser Pro
           115                 120                 125
   Val Val Val Gly Thr Thr Asp Arg Ser Gly Val Pro Thr Tyr Ser Trp
   130 135 140
   Gly Glu Asn Glu Thr Asp Val Leu Leu Leu Asn Asn Thr Arg Pro Pro
   145 150 155 160
   Gln Gly Asn Trp Phe Gly Cys Thr Trp Met Asn Ser Thr Gly Phe Thr
   165 170 175
   Lys Thr Cys Gly Gly Pro Pro Cys Asn Ile Gly Gly Val Gly Asn Asn
   180 185 190
   Thr Leu Ile Cys Pro Thr Asp Cys Phe Arg Lys His Pro Glu Ala Thr
   195 200 205
   Tyr Thr Lys Cys Gly Ser Gly Pro Trp Leu Thr Pro Arg Cys Leu Val
   210 215 220
   Asp Tyr Pro Tyr Arg Leu Trp His Tyr Pro Cys Thr Ile Asn Phe Thr
   225 230 235 240
   Ile Phe Lys Val Arg Met Tyr Val Gly Gly Val Glu His Arg Leu Asn
   245 250 255
   Ala Ala Cys Asn Trp Thr Arg Gly Glu Arg Cys Asp Leu Glu Asp Arg
   260 265 270
   Asp Arg Ser Glu Leu Ser Pro Leu Leu Leu Ser Thr Thr Glu Trp Gln
   275 280 285
<210> 3
   <211> 287
   <212> PRT
   <213> Artificial sequence
<220>
   <223> NS3 1b cloné du VHC
<400> 3
   Met Arg Gly Ser His His His His His His Gly Ser Val Asp Glu Ser
   1 5 10 15
   Met Asp Glu Phe Ala Val Asp Phe Ile Pro Val Glu Ser Met Glu Thr
   20 25 30
   Thr Met Arg Ser Pro Val Phe Thr Asp Asn Ser Ser Pro Pro Ala Val
   35 40 45
   Pro Gln Thr Phe Gln Val Ala His Leu His Ala Pro Thr Gly Ser Gly
   50 55 60
   Lys Ser Thr Lys Val Pro Ala Ala Tyr Ala Ala Gln Gly Tyr Lys Val
   65 70 75 80
   Arg Val Leu Asn Pro Ser Val Ala Ala Thr Leu Gly Phe Gly Ala Tyr
   85 90 95
   Met Ser Lys Ala His Gly Ile Glu Pro Asn Ile Arg Thr Gly Val Arg
   100 105 110
   Thr Ile Thr Thr Gly Gly Pro Ile Thr Tyr Ser Thr Tyr Gly Lys Phe
   115 120 125
   Leu Ala Asp Gly Gly Cys Ser Gly Gly Ala Tyr Asp Ile Ile Ile Cys
   130 135 140
   Asp Glu Cys His Ser Thr Asp Trp Thr Thr Ile Leu Gly Ile Gly Thr
   145 150 155 160
   Val Leu Asp Gln Ala Glu Thr Ala Gly Ala Arg Leu Val Val Leu Ala
   165 170 175
   Thr Ala Thr Pro Pro Gly Ser Ile Thr Val Pro His Pro Asn Ile Glu
   180 185 190
   Glu Val Ala Leu Ser Asn Thr Gly Glu Ile Pro Phe Tyr Gly Lys Ala
   195 200 205
   Ile Pro Ile Glu Ala Ile Lys Gly Gly Arg His Leu Ile Phe Cys His
   210 215 220
   Ser Lys Lys Lys Cys Asp Glu Leu Ala Ala Lys Leu Thr Gly Leu Gly
   225 230 235 240
   Leu Asn Ala Val Ala Tyr Tyr Arg Gly Leu Asp Val Ser Val Ile Pro
   245 250 255
   Thr Ser Gly Asp Val Val Val Val Ala Thr Asp Ala Leu Met Thr Gly
   260 265 270
   Phe Thr Gly Asp Phe Asp Ser Val Ile Asp Cys Asn Thr Cys Val
   275 280 285
<210> 4
   <211> 267
   <212> PRT
   <213> Hepatitis C virus
<400> 4
   Ala Val Asp Phe Ile Pro Val Glu Ser Met Glu Thr Thr Met Arg Ser
   1               5                   10                  15
   Pro Val Phe Thr Asp Asn Ser Ser Pro Pro Ala Val Pro Gln Thr Phe
               20                  25                  30
   Gln Val Ala His Leu His Ala Pro Thr Gly Ser Gly Lys Ser Thr Lys
           35                  40                  45
   Val Pro Ala Ala Tyr Ala Ala Gln Gly Tyr Lys Val Arg Val Leu Asn
       50                  55                  60
   Pro Ser Val Ala Ala Thr Leu Gly Phe Gly Ala Tyr Met Ser Lys Ala
   65                  70                  75                  80
   His Gly Ile Glu Pro Asn Ile Arg Thr Gly Val Arg Thr Ile Thr Thr
                   85                  90                  95
   Gly Gly Pro Ile Thr Tyr Ser Thr Tyr Gly Lys Phe Leu Ala Asp Gly
               100                 105                 110
   Gly Cys Ser Gly Gly Ala Tyr Asp Ile Ile Ile Cys Asp Glu Cys His
           115                 120                 125
   Ser Thr Asp Trp Thr Thr Ile Leu Gly Ile Gly Thr Val Leu Asp Gln
       130                 135                 140
   Ala Glu Thr Ala Gly Ala Arg Leu Val Val Leu Ala Thr Ala Thr Pro
   145                 150                 155                 160
   Pro Gly Ser Ile Thr Val Pro His Pro Asn Ile Glu Glu Val Ala Leu
                   165                 170                 175
   Ser Asn Thr Gly Glu Ile Pro Phe Tyr Gly Lys Ala Ile Pro Ile Glu
               180                 185                 190
   Ala Ile Lys Gly Gly Arg His Leu Ile Phe Cys His Ser Lys Lys Lys
           195                 200                 205
   Cys Asp Glu Leu Ala Ala Lys Leu Thr Gly Leu Gly Leu Asn Ala Val
       210                 215                 220
   Ala Tyr Tyr Arg Gly Leu Asp Val Ser Val Ile Pro Thr Ser Gly Asp
   225                 230                 235                 240
   Val Val Val Val Ala Thr Asp Ala Leu Met Thr Gly Phe Thr Gly Asp
                   245                 250                 255
   Phe Asp Ser Val Ile Asp Cys Asn Thr Cys Val
               260                 265
<210> 5
   <211> 31
   <212> PRT
   <213> Hepatitis C virus
<400> 5
   Gly Glu Gly Ala Val Gln Trp Met Asn Arg Leu Ile Ala Phe Ala Ser
   1               5                   10                  15
   Arg Gly Asn His Val Ser Pro Thr His Tyr Val Pro Glu Ser Asp
               20                  25                  30
<210> 6
   <211> 113
   <212> PRT
   <213> artificial sequence
<220>
   <223> NS5A cloné du VHC
<400> 6
   Met Ala Ser Lys Ala Thr Cys Thr Thr His His Asp Ser Pro Asp Ala
   1               5                   10                  15
   Asp Leu Ile Glu Ala Asn Leu Leu Trp Arg Gln Glu Met Gly Gly Asn
               20                  25                  30
   Ile Thr Arg Val Glu Ser Glu Asn Lys Val Val Ile Leu Asp Ser Phe
           35                  40                  45
   Asp Pro Leu Arg Ala Glu Glu Asp Glu Arg Glu Val Ser Val Ala Ala
       50                  55                  60
   Glu Ile Leu Arg Lys Ser Lys Lys Phe Pro Pro Ala Leu Pro Ile Trp
   65                  70                  75                  80
   Ala Arg Pro Asp Tyr Asn Pro Pro Leu Leu Glu Ser Trp Lys Ser Pro
                   85                  90                  95
   Asp Tyr Val Pro Pro Ala Val Met Arg Gly Ser His His His His His
               100                 105                 110
   His
<210> 7
   <211> 100
   <212> PRT
   <213> Hepatitis C virus
<400> 7
   Lys Ala Thr Cys Thr Thr His His Asp Ser Pro Asp Ala Asp Leu Ile
   1 5 10 15
   Glu Ala Asn Leu Leu Trp Arg Gln Glu Met Gly Gly Asn Ile Thr Arg
   20 25 30
   Val Glu Ser Glu Asn Lys Val Val Ile Leu Asp Ser Phe Asp Pro Leu
   35 40 45
   Arg Ala Glu Glu Asp Glu Arg Glu Val Ser Val Ala Ala Glu Ile Leu
   50 55 60
   Arg Lys Ser Lys Lys Phe Pro Pro Ala Leu Pro Ile Trp Ala Arg Pro
   65 70 75 80
   Asp Tyr Asn Pro Pro Leu Leu Glu Ser Trp Lys Ser Pro Asp Tyr Val
   85 90 95
   Pro Pro Ala Val
   100
<210> 8
   <211> 287
   <212> PRT
   <213> artificial sequence
<220>
   <223> NS3 1a cloné du VHC
<400> 8
   Met Arg Gly Ser His His His His His His Gly Ser Val Asp Glu Ser
   1 5 10 15
   Met Asp Glu Phe Ala Val Asp Phe Ile Pro Val Glu Asn Leu Glu Thr
   20 25 30
   Thr Met Arg Ser Pro Val Phe Ser Asp Asn Ser Ser Pro Pro Ala Val
   35 40 45
   Pro Gln Ser Tyr Gln Val Ala His Leu His Ala Pro Thr Gly Ser Gly
   50 55 60
   Lys Ser Thr Lys Val Pro Ala Ala Tyr Ala Ala Gln Gly Tyr Lys Val
   65 70 75 80
   Leu Val Leu Asn Pro Ser Val Ala Ala Thr Leu Gly Phe Gly Ala Tyr
   85 90 95
   Met Ser Lys Ala His Gly Ile Asp Pro Asn Ile Arg Thr Gly Val Arg
   100 105 110
   Thr Ile Thr Thr Gly Ser Pro Ile Thr Tyr Ser Thr Tyr Gly Lys Phe
   115 120 125
   Leu Ala Asp Gly Gly Cys Ser Gly Gly Ala Tyr Asp Ile Ile Ile Cys
   130 135 140
   Asp Glu Cys His Ser Thr Asp Ala Thr Ser Ile Leu Gly Ile Gly Thr
   145 150 155 160
   Val Leu Asp Gln Ala Glu Thr Ala Gly Ala Arg Leu Thr Val Leu Ala
   165 170 175
   Thr Ala Thr Pro Pro Gly Ser Val Thr Val Pro His Pro Asn Ile Glu
   180 185 190
   Glu Val Ala Leu Ser Thr Thr Gly Glu Ile Pro Phe Tyr Gly Lys Ala
   195 200 205
   Ile Pro Leu Glu Ala Ile Lys Gly Gly Arg His Leu Ile Phe Cys His
   210 215 220
   Ser Lys Lys Lys Cys Asn Glu Leu Ala Ala Lys Leu Val Ala Leu Gly
   225 230 235 240
   Val Asn Ala Val Ala Tyr Tyr Arg Gly Leu Asp Val Ser Val Ile Pro
   245 250 255
   Thr Ser Gly Asp Val Val Val Val Ala Thr Asp Ala Leu Met Thr Gly
   260 265 270
   Phe Thr Gly Asp Phe Asp Ser Val Ile Asp Cys Asn Thr Cys Val
   275 280 285
<210> 9
   <211> 267
   <212> PRT
   <213> Hepatitis C virus
<400> 9
   Ala Val Asp Phe Ile Pro Val Glu Asn Leu Glu Thr Thr Met Arg Ser
   1 5 10 15
   Pro Val Phe Ser Asp Asn Ser Ser Pro Pro Ala Val Pro Gln Ser Tyr
   20 25 30
   Gln Val Ala His Leu His Ala Pro Thr Gly Ser Gly Lys Ser Thr Lys
   35 40 45
   Val Pro Ala Ala Tyr Ala Ala Gln Gly Tyr Lys Val Leu Val Leu Asn
   50 55 60
   Pro Ser Val Ala Ala Thr Leu Gly Phe Gly Ala Tyr Met Ser Lys Ala
   65 70 75 80
   His Gly Ile Asp Pro Asn Ile Arg Thr Gly Val Arg Thr Ile Thr Thr
   85 90 95
   Gly Ser Pro Ile Thr Tyr Ser Thr Tyr Gly Lys Phe Leu Ala Asp Gly
   100 105 110
   Gly Cys Ser Gly Gly Ala Tyr Asp Ile Ile Ile Cys Asp Glu Cys His
   115 120 125
   Ser Thr Asp Ala Thr Ser Ile Leu Gly Ile Gly Thr Val Leu Asp Gln
   130 135 140
   Ala Glu Thr Ala Gly Ala Arg Leu Thr Val Leu Ala Thr Ala Thr Pro
   145 150 155 160
   Pro Gly Ser Val Thr Val Pro His Pro Asn Ile Glu Glu Val Ala Leu
   165 170 175
   Ser Thr Thr Gly Glu Ile Pro Phe Tyr Gly Lys Ala Ile Pro Leu Glu
   180 185 190
   Ala Ile Lys Gly Gly Arg His Leu Ile Phe Cys His Ser Lys Lys Lys
   195 200 205
   Cys Asn Glu Leu Ala Ala Lys Leu Val Ala Leu Gly Val Asn Ala Val
   210 215 220
   Ala Tyr Tyr Arg Gly Leu Asp Val Ser Val Ile Pro Thr Ser Gly Asp
   225 230 235 240
   Val Val Val Val Ala Thr Asp Ala Leu Met Thr Gly Phe Thr Gly Asp
   245 250 255
   Phe Asp Ser Val Ile Asp Cys Asn Thr Cys Val
   260 265
<210> 10
   <211> 5
   <212> PRT
   <213> artificial sequence
<220>
   <223> épi t ope
<400> 10
   Gln Asp Val Lys Phe
   1               5
<210> 11
   <211> 5
   <212> PRT
   <213> artificial sequence
<220>
   <223> épi t ope
<400> 11
   Gln Ile Val Gly Gly
   1               5
<210> 12
   <211> 8
   <212> PRT
   <213> artificial sequence
<220>
   <223> épi t ope
<400> 12
   Pro Arg Gly Arg Arg Gln Pro Ile
   1               5
<210> 13
   <211> 8
   <212> PRT
   <213> artificial sequence
<220>
   <223> épi t ope
<400> 13
   Gln Ile Val Gly Gly Val Tyr Leu
   1               5
<210> 14
   <211> 8
   <212> PRT
   <213> artificial sequence
<220>
   <223> épitope
<400> 14
   Arg Lys Thr Lys Arg Asn Thr Asn
   1 5
<210> 15
   <211> 6
   <212> PRT
   <213> artificial sequence
<220>
   <223> épitope
<400> 15
   Val Tyr Leu Leu Pro Arg
   1 5
<210> 16
   <211> 14
   <212> PRT
   <213> artificial sequence
<220>
   <223> épi t ope
<400> 16
   Gly Arg Thr Trp Ala Gln Pro Gly Tyr Pro Trp Pro Leu Tyr
   1 5 10

## Description

La présente invention concerne le domaine du diagnostic de l'hépatite C et en particulier un support solide utilisable dans un procédé de détection simultanée d'un antigène du virus de l'hépatite C (VHC) et d'un anticorps dirigé contre une protéine du VHC.

La première génération de tests pour la détection de l'infection par le VHC était basée sur la détection d'anticorps dirigés contre des protéines non structurales NS3/NS4 (EP 0 318 216 ; Choo et al (1989)) en utilisant en particulier une protéine c100-3 d'environ 360 acides aminés fusionnée avec la protéine superoxyde dismutase. Cette première génération ne permettait cependant de détecter que 70 à 80 % des sérums infectés par le virus.

La deuxième génération de tests, toujours basée sur la détection d'anticorps, incorporait des antigènes de plusieurs régions notamment de la protéine de Core, et des protéines NS3 et NS4 (Mimms et al (1990); EP 0 450 931). Cette deuxième génération représentait un progrès significatif puisque la sensibilité dépassait les 95% (Alter et al ((992)).

La troisième génération de tests ajoutait à ces antigènes Core, NS3et NS4 une protéine recombinante additionnelle de la région NS5 (Courouce et al (1994)).

Néanmoins, cette détection est envisagée pour les patients présentant une symptomatologie de la maladie chronique du foie du VHC et détecte mal sa phase précoce (les patients avec une suspicion d'hépatite C en phase aiguë). Par exemple ce format peut laisser passer le matériel sanguin de certains donneurs de sang contaminés dans le cadre d'une transfusion sanguine. La détection du virus lui-même est nécessaire le plus tôt possible après la contamination et avant l'apparition des anticorps. Cela est réalisé par la détection des acides nucléiques à l'aide d'une technique d'amplification type PCR (réaction de polymérase en chaîne) comme décrit par Garson et al (1990). Cette technique, malheureusement, reste complexe à mettre en oeuvre aussi bien du point de vue de la préparation d'échantillon, des risques de contamination, que de l'automatisation. Une autre approche en vue de la détection précoce de l'infection par le VHC consiste en la détection de l'antigène viral circulant de la protéine Core comme décrit par Takahashi et al (1992) bien que la quantité d'antigène Core détectable soit faible et que l'essai reste complexe à mettre en oeuvre.

Pour pallier à ces inconvénients, un essai de détection en combinaison (appelé COMBO) des antigènes du VHC et des anticorps dirigés contre le VHC représente un bon compromis pour bénéficier à la fois d'une détection précoce, par la détection de l'antigène, et du suivi de la chronicité du patient, par la détection des anticorps.

Cependant, ceci pose un problème majeur, celui de l'interférence, concernant le dosage de l'antigène Core du VHC, entre les anticorps anti-VHC présents dans le sérum et des anticorps anti-VHC marqués utilisés pour la détection de l'antigène.

Ceci est particulièrement vrai dans un système de détection simultanée, sur la même phase solide, d'anticorps anti-protéine Core du VHC et d'antigène Core du VHC. Ainsi le dépôt sur la phase solide, en vue de la détection d'anticorps anti-protéine Core du VHC, d'un antigène Core qui a les mêmes épitopes que ceux reconnus par les anticorps anti-protéine Core VHC marqués, utilisés en vue de la détection de l'antigène Core, entraîne une fixation d'anticorps marqués sur la phase solide et résulte en une réponse faussement positive de l'essai. De même, dans un processus de fabrication d'un support solide où il faut fixer à la fois un antigène Core et un anticorps anti-protéine Core, il n'est pas possible de sensibiliser la phase solide par une solution en mélange de ces deux composants sous peine de voir le blocage des sites anticorps par les antigènes utilisés pour la sensibilisation du support solide, empêchant leur réaction ultérieure avec les antigènes du sérum du patient. Ces phénomènes de compétition sont donc très handicapants pour mettre au point un test efficace.

MORADPOUR DARIUS ET AL: "Replication of hepatitis C virus" NATURE REVIEWS. MICROBIOLOGY, vol. 5, no. 6, juin 2007, pages 453-463 décrit la réplication du VHC et les fonctions de NS4B et NS5A.

WO 01/38360 divulgue l'utilisation d'un polypeptide comprenant NS4B et NS5A à des fins de diagnostic. Le polypeptide peut aussi comprendre Core et E1 ou E2. SILLANPÄÄ MAARIT ET AL: "Hepatitis C virus core, NS3, NS4B and NS5A are the major immunogenic proteins in humoral immunity in chronic HCV infection" VIROLOGY JOURNAL 2009, vol. 6, page 84 (12 pages) indique que les protéines core, NS3, NS4B et NS5A représentent les protéines antigéniques majoritaires, alors que les protéines E1, E2 et NS4A sont minoritaires.

WO 00/07023 propose un test COMBO avec à la fois la détection des antigènes Core et des anticorps anti-protéine Core dans lequel, pour éviter le problème de l'interférence, les épitopes de la protéine Core, choisis pour les différents composants du kit, sont différents. Les épitopes Core n'étant pas en nombre illimité, cette technique présente l'inconvénient d'utiliser des épitopes mineurs et peu reconnus.

Une approche semblable, mais systématisée pour combiner un maximum d'épitopes non chevauchants de la protéine Core et d'anticorps monoclonaux anti protéine Core, est décrite dans WO 2008/027942 toujours pour éviter ce problème d'interférence.

EP 1 251 353 utilise la même technique mais avec un mélange d'antigènes plus complet (NS3, NS4, NS5, Core) pour la détection des anticorps mais toujours avec ce problème d'interférence pour la protéine Core.

WO 03/095968 résout ce problème en modifiant structurellement, notamment par substitution d'acides aminés, certains épitopes cibles des antigènes utilisés pour la capture d'anticorps. Les épitopes ainsi modifiés sont alors détruits. Simultanément, les anticorps utilisés pour la capture et/ou la détection des antigènes sont eux choisis de telle sorte qu'ils reconnaissent précisément des épitopes non modifiés présents sur les antigènes du patient, et qu'ils ne puissent ainsi pas se lier aux antigènes modifiés, qui ne présentent plus ces mêmes épitopes. Puisque les épitopes ne sont plus identiques, il n'existe donc plus de compétition entre les anticorps utilisés pour capturer et/ou détecter l'antigène de VHC et les anticorps du patient. La capture de l'antigène Core et celle des anticorps anti-protéine Core pourront se faire sur une seule et même zone protéique de la protéine Core et éviteront la perte de détection d'un certain nombre d'anticorps anti-protéine Core. Une stratégie semblable est décrite dans WO 03/002749 où la protéine Core est modifiée pour que certains épitopes ne soient pas reconnus par les anticorps monoclonaux utilisés dans l'essai.

WO 01/096875 décrit un essai COMBO dans lequel ce problème d'interférence est résolu en combinant la détection de l'antigène Core du VHC et des anticorps dirigés contre les épitopes discontinus présents dans la protéine de 680 acides aminés (aa) de NS3/NS4a. Mais les résultats en sensibilité/spécificité sont très limités dans cette demande et il n'est pas démontré que le niveau de sensibilité soit suffisant pour un test de détection.

Il existe donc un besoin pour un test de détection du VHC combiné antigène et anticorps qui soit sensible et spécifique, le plus simple possible et qui évite le problème de l'interférence entre les antigènes et les anticorps dirigés contre la protéine Core.

A cette fin, il a été trouvé un support solide de test immunologique pour la détection du VHC sur lequel est fixé :
a) au moins un anticorps dirigé contre la protéine Core du VHC,
b) et au moins un polypeptide comprenant au moins un épitope de la protéine E2 du VHC et au moins un épitope d'une protéine choisie parmi les protéines E1, NS3, NS4, NS5 du VHC,
et sur lequel ledit au moins un polypeptide ne comprend pas un épitope de la protéine Core du VHC.

En particulier sur le support solide de test immunologique pour la détection du VHC est fixé :
a) au moins un anticorps dirigé contre la protéine de Core du VHC,
b) et au moins un polypeptide comprenant au moins un épitope de la protéine E2 du VHC,
c) et au moins un polypeptide comprenant au moins un épitope d'une protéine choisie parmi les protéines E1, NS3, NS4, NS5 du VHC,
et sur lequel ledit au moins un polypeptide ne comprend pas un épitope de la protéine de Core du VHC.

L'invention propose en outre un procédé de détection *in vitro* d'une infection par le VHC dans un échantillon biologique comprenant la détection d'au moins un antigène du VHC et d'un anticorps dirigé contre le VHC présent dans l'échantillon biologique et dans lequel
on dispose d'un support tel que décrit ci-dessus,
on incube ledit support avec l'échantillon biologique dans des conditions permettant le formation de complexes antigènes-anticorps,
on révèle les complexes antigènes-anticorps formés.

De manière avantageuse, le support solide selon l'invention comprend ou consiste en a) au moins un anticorps dirigé contre la protéine Core du VHC, b) au moins un polypeptide comprenant ou consistant en au moins un épitope de la protéine E2 du VHC et c) au moins un polypeptide comprenant ou consistant en au moins un épitope d'une protéine choisie parmi les protéines NS3, NS4, NS5 du VHC, de préférence NS4B et NS5A.

De manière plus avantageuse, le support solide selon l'invention comprend ou consiste en a) au moins un anticorps dirigé contre la protéine Core du VHC, b) au moins un polypeptide comprenant ou consistant en au moins un épitope de la protéine E2 du VHC et c) au moins un polypeptide comprenant ou consistant en au moins un épitope de NS3 du VHC et au moins un épitope d'une protéine choisie parmi les protéines NS4, NS5 du VHC.

De manière préférentielle, le support solide selon l'invention comprend ou consiste en a) au moins un anticorps dirigé contre la protéine Core du VHC, b) au moins un polypeptide comprenant ou consistant en au moins un épitope de la protéine E2 du VHC et c) au moins un polypeptide comprenant ou consistant en au moins un épitope de NS3 du VHC et au moins un épitope d'une protéine choisie parmi les protéines NS4B et NS5A du VHC.

Dans une autre forme de réalisation de l'invention, le support solide selon l'invention comprend ou consiste en a) au moins un anticorps dirigé contre la protéine Core du VHC, b) au moins un polypeptide comprenant ou consistant en au moins un épitope de la protéine E2 du VHC et c) au moins un polypeptide comprenant ou consistant en au moins un épitope d'une protéine NS4B du VHC et au moins un épitope d'une protéine NS5A du VHC.

Dans tous les cas, aucun polypeptide comprenant un épitope de la protéine Core du VHC n'est fixé sur le support solide.

Le terme VHC (virus de l'hépatite C) couvre toutes les souches, types, sous types et génotypes du virus responsable de l'hépatite C. Ceci inclut les 6 génotypes principaux 1, 2 ,3 ,4, 5, 6 ainsi que leurs sous types 1a, 1b, etc.

Les régions du VHC sont définies approximativement par la numérotation employée dans Choo QL et al 1991 et sont résumées dans le tableau ci-dessous pour le VHC 1 :

| Domaine | Position en aa acides aminés |
|---|---|
| Core | 1-191 |
| E1 | 192-383 |
| E2 | 384-746 |
| P7 | 747-809 |
| NS2 | 810-1026 |
| NS3 | 1027-1657 |
| NS4A | 1658-1711 |
| NS4B | 1712-1972 |
| NS5A | 1973-2420 |
| NS5B | 2421-3011 |

Le terme polypeptide (ou antigène) se réfère à un polymère d'acides aminés et n'est pas limité à un nombre minimum d'acides aminés ou à une taille particulière. Les acides aminés peuvent être naturels (les 20 codant pour une protéine) ou synthétiques comme l'ornithine ou l'acide γ-amino butyrique ou modifiés par glycosylation, acétylation, phosphorylation ou équivalent de telle manière que le polypeptide conserve l'activité souhaitée notamment antigénique vis-à-vis d'anticorps produits par un patient contre le VHC.

Le terme épitope se réfère à une séquence d'au moins 3, 4 ou 5 acides aminés, en particulier entre 6-8 et 12-15 acides aminés, qui se lie à un anticorps. Il n'y a pas de taille supérieure critique pour un épitope. La séquence d'un épitope peut comporter des modifications dites conservatrices qui ne changent pas significativement la liaison entre l'épitope et l'anticorps d'un point de vue de la spécificité.

Par l'expression « au moins un épitope de la protéine X », on entend la protéine X ou un ou plusieurs de ses épitopes. Ainsi, par exemple, par l'expression « au moins un épitope de la protéine E2 », on entend la protéine E2 elle-même ou un ou plusieurs de ses épitopes.

Aussi, selon un mode de réalisation préféré de l'invention, sur le support solide de test immunologique pour la détection du VHC sont fixés :
a) au moins un anticorps dirigé contre la protéine de Core du VHC et
b) un polypeptide consistant en (i) un peptide de la protéine E2 du VHC choisi parmi la protéine E2 elle-même et un ou plusieurs de ses épitopes et (ii) un peptide des protéines E1, NS4B et/ou NS5A du VHC choisi parmi les protéines elles-mêmes et un ou plusieurs de leurs épitopes, et le cas échéant également en (iii) un peptide de la protéine NS3 choisi parmi la protéine elle-même et un ou plusieurs de ses épitopes.

De préférence, le peptide (ii) est choisi parmi les protéines NS4B, NS5A et un ou plusieurs de leurs épitopes. De préférence encore, le peptide (iii) n'est pas présent dans le polypeptide b).

Selon un autre mode de réalisation de l'invention, sur le support solide de test immunologique pour la détection du VHC sont fixés :
a) au moins un anticorps dirigé contre la protéine de Core du VHC,
b) un peptide de la protéine E2 du VHC choisi parmi la protéine E2 elle-même ou un ou plusieurs de ses épitopes et
c) un peptide des protéines E1, NS4B et/ou NS5A du VHC choisi parmi les protéines elles-mêmes et un ou plusieurs de leurs épitopes, avec, le cas échéant
d) un peptide de la protéine NS3 choisi parmi la protéine elle-même et un ou plusieurs de ses épitopes.

De préférence, le peptide c) est choisi parmi les protéines NS4B, NS5A et un ou plusieurs de leurs épitopes. De préférence encore, le support ne comprend pas de peptide d).

Un polypeptide fixé sur le support solide peut comporter plusieurs épitopes de protéines différentes voire de sous types différents. Les constructions de ce type (Multiple Epitopes Fusion Antigens : MEFA) sont bien décrites dans WO 01/096875 ou WO 97/44469.

De préférence, le polypeptide comprenant au moins un épitope de la protéine E2 sera différent du polypeptide comprenant au moins un épitope de E1 et/ou NS4 et/ ou NS5.

Par protéine E2, on entend une glycoprotéine d'enveloppe d'un poids moléculaire d'environ 70-72kd. Le terme protéine E2 inclut aussi les mutants et protéines tronquées qui ont un comportement immunologique similaire avec la protéine E2 (même réactivité croisée entre les deux avec un anticorps de référence). Par exemple une forme de E2 décrite dans l'art antérieur s'étend de la position 384 à 746 mais une autre forme de E2 s'étend jusqu'à la positon 809 et une protéine E2 tronquée au delà de la position 683 est décrite dans cette demande. Des insertions ont été décrites entre les acides aminés 383 et 384 de même que des délétions entre les positions 384-387 (Kato et al 1992). De préférence, la protéine E2 est tronquée d'au moins 10, 30, 50, 60, 63, 70, 80, 90, 126 acides aminés de sa partie C terminale.

Le terme anticorps se réfère à tout anticorps entier ou fragment fonctionnel d'un anticorps comprenant ou consistant en au moins un site de combinaison antigénique, permettant audit anticorps de se lier à au moins un épitope d'un composé antigénique. A titre d'exemple de fragments d'anticorps on peut citer les fragments Fab, Fab', F(ab')₂ ainsi que les chaînes scFv (Single chain variable fragment), dsFv (Double-stranded variable fragment). Ces fragments fonctionnels peuvent notamment être obtenus par génie génétique.

Par « au moins un anticorps dirigé contre la protéine Core », on entend un ou plusieurs anticorps anti-protéine Core.

Les anticorps selon l'invention sont soit des anticorps polyclonaux, soit monoclonaux.

Les anticorps polyclonaux sus-mentionnés peuvent être obtenus par immunisation d'un animal avec au moins un antigène d'intérêt, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment un antigène d'intérêt.

Les anticorps monoclonaux peuvent être obtenus par la technique des hybridomes dont le principe général est rappelé ci-après : dans un premier temps, on immunise un animal, généralement une souris, (ou des cellules en culture dans le cadre d'immunisations *in vitro*) avec un antigène d'intérêt, dont les lymphocytes B sont alors capables de produire des anticorps contre ledit antigène. Ces lymphocytes producteurs d'anticorps sont ensuite fusionnés avec des cellules myélomateuses "immortelles" pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de l'antigène d'intérêt pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité.

Dans le contexte de l'invention, un échantillon biologique est de préférence constitué par un fluide biologique tel que sérum, plasma, sang, sang total mais aussi urine, tissu, liquide céphalo-rachidien ou autre. L'échantillon biologique peut être traité dans une étape préalable ou mis en présence de la phase solide dans des conditions, par exemple des conditions acides, favorisant l'exposition des antigènes à détecter. Des détergents de type ioniques ou non ioniques par exemple le triton X100 ou le SDS (sodium dodecyl sulfate) ou des dérivés de poly(oxyethylène) comme le NP40 peuvent être utilisés.

Les polypeptides de l'invention sont produits par les techniques connues en soi par l'homme du métier en utilisant les techniques standards de biologie moléculaire et de génie génétique, ou de manière chimique dans le cas de peptide de plus petite taille par exemple par synthèse sur phase solide. Toutes ces techniques sont bien connues de l'homme du métier. Les régions épitopiques d'un antigène peuvent être déterminés par les techniques dites de cartographie d'épitopes comme décrit dans Methods in Molecular Biology, Epitopes Mapping Protocols, vol 66, GE. Morris ed., 1996, Humana Press.

Par marquage, on entend la fixation d'un marqueur capable de générer directement ou indirectement un signal détectable. Une liste non limitative de ces marqueurs consiste en : les enzymes qui produisent un signal détectable par exemple par colorimétrie, fluorescence, luminescence, comme la peroxydase de raifort, la phosphatase alcaline, la α-galactosidase, la glucose-6-phosphate déshydrogénase ; les chromophores comme les composés fluorescents, luminescents, colorants, les molécules radioactives comme le ³²P, le ³⁵S ou le ¹²⁵I, et les molécules fluorescentes telles que la rhodamine ou les phycocyanines. Des systèmes indirects peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide. Dans ce cas, c'est le ligand qui est fixé sur le polypeptide ou l'anticorps. L'anti-ligand peut être détectable directement par les marqueurs décrits au paragraphe précédent ou être lui-même détectable par un ligand/anti-ligand.

Le terme support solide tel qu'utilisé ici inclut tous les matériaux sur lesquels peut être immobilisé un antigène et/ou un anticorps pour une utilisation dans des tests diagnostiques. Des matériaux naturels, de synthèse, modifiés ou non chimiquement, peuvent être utilisés comme support solide, notamment des polymères tels que polychlorure de vinyle, polyéthylène, polystyrènes, polyacrylate, polyamide, ou copolymères à base de monomères vinyl aromatiques, esters d'acides carboxyliques insaturés, chlorure de vinylidène, diènes ou composés présentant des fonctions nitrile (acrylonitrile); des polymères de chlorure de vinyle et de propylène, polymère de chlorure de vinyle et acétate de vinyle; copolymères à base de styrènes ou dérivés substitués du styrène; des fibres synthétiques telles que le nylon; des matériaux inorganiques tels que la silice, le verre, la céramique, le quartz; des latex, des particules magnétiques; des dérivés métalliques. Le support solide selon l'invention peut être, sans limitation, sous la forme d'une plaque de microtitration, d'une feuille, d'un cône, d'un tube, d'un puits, de billes, particules ou analogues, support plan comme un wafer de silice ou silicium.

La fixation du ou des antigènes et anticorps sur le support solide peut se réaliser par tout moyen direct ou indirect en particulier par adsorption passive. La fixation des antigènes et anticorps peut se réaliser en mélange ou séquentiellement ou une combinaison des deux.

Dans un mode de réalisation particulier, tous les antigènes et anticorps de l'invention seront fixés sur la même zone par exemple dans un puits d'une plaque de microtitration.

Dans un autre mode de réalisation les antigènes et les anticorps seront fixés dans des zones discrètes et donc différentes du support solide comme par exemple sur un cône du VIDAS (bioMérieux, Marcy l'Etoile), par exemple comme mis en oeuvre dans le kit HIV DUO.

La présence des anticorps et antigènes dans l'échantillon biologique est révélée par des moyens de détections. S'agissant de la détection de l'antigène, l'invention prévoit notamment une détection à l'aide d'au moins un anticorps de détection. Un tel anticorps de détection, marqué, est capable de se lier à l'antigène capturé, par liaison affine, en reconnaissant un site épitopique, différent de celui reconnu par l'anticorps de capture, ou identique du fait de la présence de motif répétitif au niveau de l'antigène. S'agissant de la détection des anticorps, on peut utiliser notamment des anticorps anti-immunoglobuline, ou anti-isotype, marqués, par exemple des anti-Immunoglobulines G ou des antigènes marqués. Toutes ces techniques de détection sont bien connues de l'homme du métier.

Le support solide selon l'invention comprend au moins 1 anticorps dirigé contre la protéine Core, de préférence au moins 2 anticorps dirigés contre la protéine Core. Dans un mode de réalisation préférentiel, au moins un de ces anticorps reconnaît un épitope choisi parmi les positions en acides aminés 2 à 120 de ladite protéine Core, particulièrement 15 à 90, avantageusement les épitopes 20-24 (SEQ ID N° 10 : QDVKF); 29-33 (SEQ ID N° 11 : QIVGG); 58-65 (SEQ ID N° 12 : PRGRRQPI); 29-37 (SEQ ID N° 13 : QIVGGVYL); 7-17 (SEQ ID N° 14 : RKTKRNTN); 34-39 (SEQ ID N° 15 : VYLLPR) ; 73-86 (SEQ ID N° 16 : GRTWAQPGYPWPLY) comme défini dans C. Jolivet-Reynaud et al (1998). Le fait de ne pas avoir de problème de compétition avec le support solide selon l'invention de part l'absence de l'antigène Core sur ledit support permet de multiplier le nombre d'anticorps anti-Core fixés sur le support solide pour améliorer la sensibilité ou la spécificité. On peut donc avoir au moins 2 ou au moins 3 anticorps fixés sur le support solide.

Dans une combinaison particulière selon l'invention, sur le support solide pour la partie détection d'anticorps, est fixé au moins un polypeptide qui comprend au moins 10, avantageusement 12, de préférence 15 acides aminés contiguës de la séquence SEQ ID n° 2 et/ou la séquence SEQ ID n° 4 et/ou la séquence SEQ ID n°5 et/ou la séquence SEQ ID n°7 et/ou SED ID n° 9.

Selon un mode de réalisation particulier, le support de l'invention remplit au moins l'une des caractéristiques suivantes :
- le peptide (ii) comprend au moins 10 acides aminés contiguës de la séquence SEQ ID n°5 et/ou la SEQ ID n°7 et
- le peptide (i) comprend au moins 10 acides aminés contiguës de la séquence SEQ ID n° 2

Dans une autre combinaison particulière selon l'invention sur le support solide, sont fixés au moins 3 polypeptides différents comprenant au moins 10, avantageusement 12, de préférence 15 acides aminés contiguës de la séquence SEQ ID n° 2 pour un premier polypeptide, de la séquence SEQ ID n° 4 pour un deuxième polypeptide et de la séquence SEQ ID n°5 et/ou SEQ ID N°7 pour le troisième.

Selon un mode de réalisation particulier, le support de l'invention remplit au moins l'une des caractéristiques suivantes :
- le peptide c) comprend au moins 10 acides aminés contiguës de la séquence SEQ ID n°5 et/ou la SEQ ID n°7 et
- le peptide b) comprend au moins 10 acides aminés contiguës de la séquence SEQ ID n° 2.

Cet immunoessai combiné peut être réalisé selon divers formats bien connus de l'homme du métier : en phase solide ou en phase homogène ; en un temps ou en deux temps ; en méthode double sandwich (sandwich pour les deux détections d'antigènes et d'anticorps) ; ou en méthode indirecte (pour la détection d'anticorps) combinée à une méthode sandwich (pour la détection d'antigène), à titre d'exemples non limitatifs.

Un format d'immunoessai de type sandwich entre deux anticorps (de capture et de détection) est particulièrement avantageux pour la détection des antigènes présents dans l'échantillon biologique, tandis que les anticorps peuvent être révélés en mettant en oeuvre un antigène de capture et un conjugué marqué qui se fixe sur l'anticorps (selon un format communément désigné comme format indirect).

Un format d'immunoessai de détection des antigènes par compétition est également possible. D'autres modalités d'immunoessai sont encore envisageables et bien connues de l'homme du métier.

La détection simultanée d'antigène du VHC et d'anticorps dirigé contre le VHC microorganisme peut être réalisée en un seul temps, à savoir en mettant simultanément en présence l'échantillon biologique, les moyens de détection, tels que notamment le ou les anticorps de détection, en même temps que le ou les anticorps de capture et le ou les antigène(s) de capture. Dans ce cas, l'immunoessai de détection de l'antigène et l'immunoessai de détection des anticorps sont tous les deux réalisés de préférence en sandwich. De manière alternative, les moyens de détection, tels que notamment le ou les anticorps de détection, peuvent être ajoutés au mélange dans un deuxième temps, c'est à dire après que les premiers complexes antigènes-anticorps se sont formés. On parle alors de dosage en deux temps.

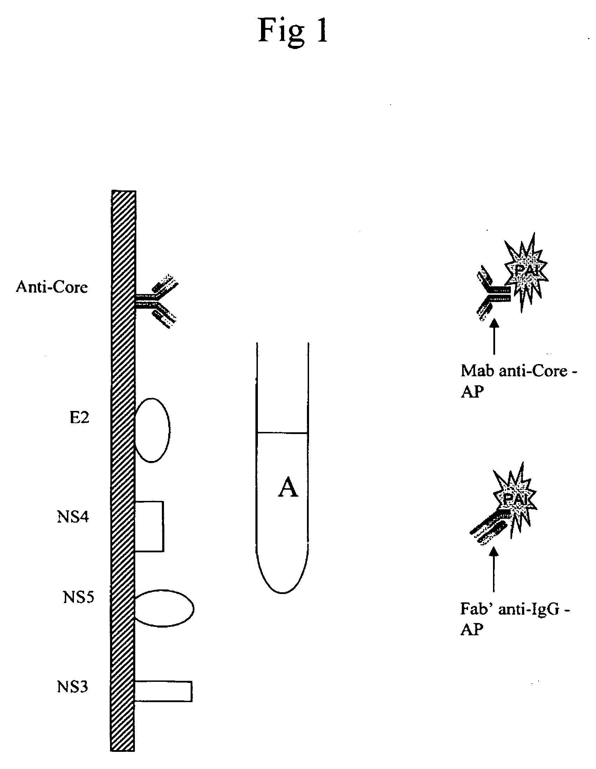

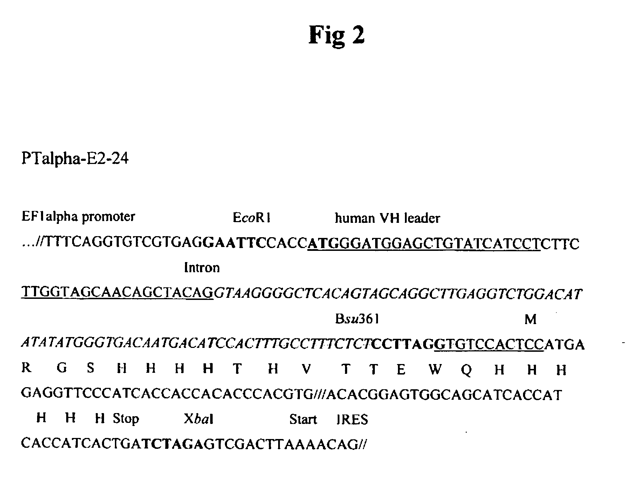

L'invention sera mieux comprise à l'aide des exemples suivants donnés à titre illustratif et non limitatif, ainsi qu'à l'aide des figures 1 et 2 annexées sur lesquelles :

### Figures :

La figure 1 représente un exemple de test ELISA utilisant un support solide selon l'invention sur lequel est fixé un anticorps anti-protéine Core ainsi que 4 antigènes du VHC respectivement E2, NS3, NS4 et NS5. Un échantillon biologique de patient A est incubé au contact de ce support solide. Cet échantillon, qui peut être du sérum, contient potentiellement des anticorps anti-VHC en particulier anti-E2 et/ou NS3 et/ou NS4 et/ou NS5 et des antigènes du virus en particulier des antigènes de Core susceptibles de réagir avec les réactifs biologiques présents sur le support solide. La révélation de la fixation de ces anticorps et antigènes du prélèvement est réalisée avec un anticorps monoclonal anti-Core lié à la phosphatase alcaline (AP) et un anticorps monoclonal (sur la figure un fragment de type Fab') anti IgG humaine lui aussi marqué la phosphatase alcaline.
La figure 2 représente la composition en séquence du clone PTalpha-E2-24 : le promoteur EF1 alpha est suivi par le site de restriction EcoRI et la région contenant la séquence de la chaîne lourde de la région variable de l'immunoglobuline G (VH leader, séquence soulignée) interrompue par une séquence intron (séquence en italique). Le clonage du gène codant pour la protéine E2 (H384 à Q673) contenant les tags (Maximilian: MRGSHHH et His₆: HHHHHH) a été réalisé après ligation du fragment PCR digéré par B*su*361 et X*ba*I.

### Exemple 1 : Matériel utilisé

Les séquences protéiques sont indiquées selon la nomenclature du code à une lettre pour les acides aminés, de N terminal vers le C terminal.

### 1.1 Anticorps monoclonaux anti-protéine Core

Les anticorps monoclonaux ont été obtenus après l'immunisation des souris femelles BALB/c JYco de 4 à 6 semaines d'âge (IFFA Credo, Les Oncins, L'Arbresle, France). Les souris ont été immunisées par la voie intra-péritonéal. Le protocole consistait en sept injections de la protéine VHC Core purifiée (10 microgrammes/ml) réalisées à deux semaines d'intervalles. Les injections de protéines sont réalisées avec l'adjuvant de Freund complet pour la première et incomplet pour les suivantes. Quatre jours après la dernière injection, les cellules de la rate ont été sélectionnées et fusionnées d'après le protocole proposé par Köhler et Milstein (1975, 1976) avec les cellules myéloïdes de souris Sp2/0-Ag14. Après 12 à 14 jours, les surnageant de culture cellulaire ont été évalués par un test immunoenzymatique (ELISA), dans lequel le même antigène utilisé pour les immunisations a été déposé en phase solide. Les clones positifs sécrétant des anticorps anti-protéine Core ont été sous clonés deux fois par la méthode de dilution limite. Les ascites ont été obtenus des souris après l'injection intra-péritonéal de 0,5 ml de Pristane et de 10⁶ cellules d'hybridome. Les anticorps monoclonaux IgG anti-protéine Core ont été purifiés en colonne de protéine-A Sepharose 4FF selon les instructions de Pharmacia. Les anticorps monoclonaux purifiés ont été biotinylés avec le réactif Sulfo-NHS-LC-Biotin (Merck, Rockford, IL) d'après Gretch et al (1987) pour les tests de détermination antigénique.

Une fois les anticorps disponibles, il a été possible de procéder à l'identification des épitopes reconnus sur la protéine Core du VHC. Les résultats de l'identification des épitopes spécifiques par 9 anticorps monoclonaux obtenus anti-protéine Core ont été publiés par Jolivet-Reynaud et al (1998). Plusieurs anticorps ont été sélectionnés par ce procédé et notamment le 19D9D6, le 7G9B8 et le 7G12A8. L'anticorps monoclonal 19D9D6 appartient au Group I et reconnaît les 45 premiers acides animés de la protéine VHC Core, plus précisément la région comprenant les acides animés 25 à 45. D'après la structure en cristal du site antigénique immunodominant de la protéine VHC Core complexé à l'anticorps monoclonal 19D9D6, il apparaît que l'épitope minimal linéaire reconnu est le QIVGGVYLL situé entre les acides aminés 29-37 (Ménez et al (2003)).

### 1.2 Protéine E2

Pour procéder aux tests ELISA, il a été nécessaire de produire et de purifier la protéine d'enveloppe E2.

Le fragment d'ADN codant pour l'ectodomaine de l'enveloppe 2 (aa 384-673 du génome VHC selon Choo at al 1991) a été amplifié à partir de la souche VHC-JA (génotype 1b, plasmide pCMV-C980) par PCR et cloné dans le vecteur d'expression stable pT-alpha. Le gène de l'enveloppe est sous le contrôle du promoteur humain EF1 et du Poliovirus humain IRES (« Internal Ribosome Entry ») dans lequel est contenu le gène DHFR (Dihydrofolate reductase) de souris (marqueur de sélection). Deux séquences additionnelles signatures « tags » ont été introduites lors du clonage: la première nommée Maximilian tag (MRGSHHH séquence en acides aminés (aa) représentée par le code à une lettre) située en 5' du gène N terminal de la protéine traduite et la deuxième His₆-tag (HHHHHH) située en 3'. La construction VHC E2 a été contrôlée par séquençage et son expression a été vérifiée par immunoblot (anticorps anti-Maximilian tag et anti-His₆-tag) après transfection. La transfection des cellules CHO (« Chinese Hamster Ovarian ») DHFR- a été effectuée par électroporation du plasmide pT-alpha E2. Le milieu sans nucléoside a été utilisé pour la sélection des clones positifs. L'amplification des clones a été réalisée afin d'augmenter le nombre de copies du gène codant pour l'enveloppe 2 intégrée dans le plasmide à l'aide d'une concentration croissante de methotrexate (MTX) : 20 nM, 100 nM, 250 nM, 500 nM, 1 micromole, 2 micromoles, 4 micromoles et 8 micomoles. La lignée cellulaire exprimant la protéine E2 a été sous-clonée et le clone, appelé PTalpha-E2-24, a été sélectionné. La composition de ce clone est décrite dans la figure 2. D'autres systèmes d'expression eucaryote type COS sont utilisables pour exprimer cette protéine E2.

Une fois le clone E2 amplifié, un protocole de purification a été mis au point à l'aide des tags histidine. Les tags histidine permettent de purifier la protéine VHC E2 avec une résine métal-chelate, telle que par exemple Ni-NTA de la société Qiagen, directement à partir du surnageant de culture. Les quantités de VHC E2 récupérées après élution indiquent que la capacité de fixation maximum théorique de la colonne est atteinte. Notons qu'aucun signal n'est détecté dans le tampon de lavage après passage sur la colonne. Alternativement, les gels d'électrophorèse ont fait l'objet d'une coloration avec le réactif Blue-PAGE (Fermentas) afin d'évaluer le degré de pureté de la protéine purifiée. Dans l'échantillon final purifié et concentré, seule la protéine VHC E2 est détectée après coloration. Le degré de pureté est donc estimé à ≥ 95%. Enfin, les échantillons obtenus ont été dosés avec le kit micro-BCA (Pierce). La méthode de production et de purification nous permet d'obtenir de façon reproductible 5 mg de protéine par litre de surnageant.

Lors de l'expression du clone PTalpha-E2-24 en système CHO, la séquence de E2 exprimée est la suivante SEQ ID n° 1 :

La partie en gras représente les acides aminés apportés dans la séquence par le plasmide.

La séquence sans ces acides aminés du plasmide est indiquée par la SEQ ID n°2.

### 1.3 Protéine NS3

Le gène codant pour la protéine NS3 domaine hélicase (acides aminés : 1192-1458 selon Choo QL et al (1991)), génotype 1b, a été cloné dans le vecteur d'expression pMR80 (Cheynet et al, 1993) en fusion avec 6 histidines situé en position 5' du gène NS3 domaine hélicase, comme décrit précédemment (Arribillaga et al., 2002). Brièvement, la souche bactérienne d' *Escherichia coli* JM109 a été ensemencée dans 50 ml de milieu LB (Bacto-Tryptone 10g/l ; Extrait de levure 5g/l, NaCl 10g/l) additionné de 100microg/ml d'ampicilline et cultivée toute la nuit à 37°C. Le lendemain, la culture de la souche bactérienne recombinante a été dilué au 1/50 et cultivée à 37°C jusqu'à que la densité optique (DO₆₀₀) atteigne 0.6. L'induction de l'expression de la protéine NS3 domaine hélicase a été réalisée après l'addition de 1mM Isopropyl-beta-D-thiogalactopyranoside (IPTG) (Gibco/BRL), durant 3 à 4 heures à 30°C avec une agitation de 250 rpm (unités de rotation). Après ce temps, le culot bactérien est repris dans le tampon de lyse et soumis à une sonication, puis centrifugé à 25 000 g pendant 30 min. La fraction soluble a été utilisée pour la purification sur colonne Ni-NTA Agarose (Qiagen) en suivant les instructions du fabricant. La protéine recombinante NS3 domaine helicase a été purifiée avec 300mM d'Imidazole et dialysée toute la nuit en PBS. La protéine a été analysée en gel de bleu de Coomassie après électrophorèse en gel SDS à 12% et en spectromètre de masse par analyse en MALDI-TOF avec l'instrument Voyager DE-PRO.

La séquence en acide aminé de la protéine NS3 génotype 1b exprimée est la SEQ ID N°3:

La partie en gras représente les acides aminés apportés dans la séquence par le plasmide. La séquence de NS3 sans ces acides aminés du plasmide est indiquée par la SEQ ID n°4.

De la même façon, une protéine NS3 domaine hélicase mais correspondant au génotype la est préparée suivant le même mode opératoire.

La séquence en acide aminé de la protéine NS3 génotype 1a exprimée est la SEQ ID N°8 :

La partie en gras représente les acides aminés apportés dans la séquence par le plasmide. La séquence de NS3 génotype 1a sans les acides aminés apportés par le plasmide est indiquée par la SEQ ID n°9.

### 1.4 Protéine NS4B

Le peptide synthétique préparé selon les méthodes usuelles en phase solide et issu de NS4B génotype 1b comprend les 31 acides-aminés des positions 1909 à 1939 relativement à la numérotation de la souche HCV-1 (Choo et al (1991). La séquence est la SEQ ID n° 5 : GEGAVQWMNRLIAFASRGNHVSPTHYVPESD.

### 1.5 Protéine NS5A

Le gène codant pour la protéine NS5A (acides aminés: 2212 à 2311 selon Choo Q.L. et al (1991)), génotype 1b, a été cloné dans un plasmide pET21b et exprimés en *E. coli* BL21 en fusion avec 6 histidines situées en position 3' du gène NS5. Brièvement, la souche bactérienne d' *Escherichia coli* JM109 a été ensemencée dans 50 ml de milieu LB (Bacto-Tryptone 10g/l ; Extrait de levure 5g/l, NaCl 10g/l additionné de 100 microgramme/ml d'ampicilline et cultivée toute la nuit à 37°C. Le lendemain, la culture de la souche bactérienne recombinante a été dilué au 1/50 et cultivée à 37°C jusqu'à que la densité optique (DO₆₀₀) atteigne 0,6. L'induction de l'expression de la protéine NS5 a été réalisée après l'addition de 0,4 mM Isopropyl-beta-D-thiogalactopyranoside (IPTG) (Gibco/BRL), durant 3 à 4 heures à 30°C avec une agitation de 250 rpm. Après ce temps, le culot bactérien est repris dans le tampon de lyse et soumis à une sonication, puis centrifugé à 25 000 g pendant 30 min. La fraction soluble a été utilisée pour la purification sur colonne Ni-NTA Agarose (Qiagen) en suivant les instructions proposées par le fabricant. La protéine recombinante NS5 a été purifiée avec 300mM d'Imidazole et dialysée toute la nuit en PBS. La protéine a été analysée par électrophorèse sur gel SDS à 12% coloré en bleu de Coomassie et par spectrométrie de masse MALDI-TOF (Voyager DE-PRO).

La séquence en acide aminé de la protéine NS5A exprimée est la SEQ ID N°6:

La partie en gras représente les acides aminés apportés dans la séquence par le plasmide.

La séquence de NS5A sans ces acides aminés apportés par le plasmide est indiquée par la SEQ ID n°7.

### Exemple 2 : Format des tests

L'essai peut se faire en microplaques 96 puits soit avec les antigènes séparés en puits individuels, soit en mélange dans un seul puits.

### 2.1 Format de l'essai avec les antigènes séparés

### 1. Sensibilisation (« coating ») des puits

### a. Anticorps anti-protéine Core

Les puits sont sensibilisés pendant 2 heures à 37°C sur microplaques MaxiSorb 96 puits (Nunc), avec 200 microlitres d'une solution de l'anticorps monoclonal de souris anti-protéine Core 19D9D6 dilué à 4 microgrammes/ml dans le tampon TBS (Tris tamponné avec une solution saline). Les plaques sont lavées 3 fois avec 300 microlitres de TBS-Tween 20 0,05% (tampon de lavage) puits passivées pendant une nuit à température ambiante dans un tampon TBS-Tween 20 0,05%, BSA 20g/l (Sigma), IgG souris 0,1g/l (Scantibodies laboratory) (tampon de passivation). Les plaques sont lavées 3 fois avec 300 microlitres en tampon de lavage.

### b. Protéine E2

Les puits sont sensibilisés pendant 2 heures à 37° sur microplaques MaxiSorb 96 puits (Nunc), avec 200 microlitres d'une solution de l'antigène dilué à 1 microgramme/ml dans le tampon TBS. Les plaques sont lavées 3 fois avec 300 microlitres de TBS-Tween 20 0,05% (tampon de lavage) puits passivées pendant une nuit à température ambiante dans un tampon TBS-Tween 20 0,05%, BSA 20g/l (Sigma), IgG souris 0,1g/l (Scantibodies laboratory). Les plaques sont lavées 3 fois avec 300 microlitres en tampon de lavage.

### c. NS3, NS4B et NS5A

Le protocole pour chaque protéine est identique à celui décrit précédemment pour la protéine E2.

### 2. Incubation de l'échantillon

Les puits sont incubés avec 200 microlitres d'échantillon dilués au 1/10 en tampon de passivation pendant 1 heure à 37° puis lavés 5 fois avec 300 microlitres de tampon de lavage.

### 3. Incubation des conjugués

### a. Conjugué pour la détection de la protéine Core.

200 microlitres d'une solution de l'anticorps monoclonal de souris anti-protéine Core 19D9D6 conjugué à la phosphatase alcaline dilué à 0,1 microgramme/ml en tampon de passivation est incubé pendant 1 heure à 37°. Les puits sont lavés 5 fois avec 300 microlitres de tampon de lavage.

### b. Conjugué pour la détection des anticorps du sérum

Les puits sensibilisés avec les antigènes E2, NS3, NS4B ou NS5A sont incubés pendant 1 heure à 37° avec 200 microlitres d'une solution de Fab' anti-IgG humaines conjugué avec la phosphatase alcaline dilué à 0,05 microgramme/ml en tampon de passivation. Les puits sont lavés 5 fois avec 300 microlitres de tampon de lavage.

### 4. Révélation

La réaction est révélée avec 200 microlitres d'un substrat pNPP (Sigma) pendant 30 min à température ambiante et stoppée par ajout de 50 microlitres de NaOH 1M. Le signal est lu sur un lecteur de microplaques à 405nm. La valeur seuil est calculée à partir du signal donné par la moyenne de 3 échantillons négatifs + 3 fois l'écart-type de ce signal. Le résultat peut être exprimé en ratio signal/seuil (s/co); un s/co supérieur à 1 est interprété comme positif.

### 2.2 Résultats de l'essai avec les antigènes séparés :

La précocité de la détection en utilisant le format de test avec les antigènes séparés est évaluée avec 10 panels de séroconversion de patients VHC fournis par Zeptometrix Corp (panel HCV 9044, 6212, 6213, 6214, 6215 et 6227) et Seracare BBI diagnostics (panels PHV908, PHV(910(M), PHV911 et PHV917(M)). Les échantillons sont des plasmas issus de prélèvements longitudinaux de personnes infectés par le VHC. Les résultats sont exprimés en Signal/Seuil; les valeurs supérieures à 1 sont considérées positives.

Les panels PHV911, 9044, 6212, 6213 et 6215 sont testés avec la protéine NS3 génotype la et les panels PHV908, PHV910(M), PHV917(M), 6214 et 6227 avec la protéine NS3 génotype 1b.

La première colonne représente un sérum du panel avec son numéro d'identification ID

Chaque antigène (E2, NS3, NS4B, NS5A) est mesuré individuellement et est représenté dans les colonnes suivantes. La dernière colonne représente les résultats en détection d'antigènes avec l'anticorps 19D9D6.

**Tableau 1 :**

| **ID** | **E2** | **NS3** | **NS4B** | **NS5A** | **19D9D6** |
|---|---|---|---|---|---|
| PHV908-1 | 0,5 | 0,7 | 0,6 | 0,3 | 0,1 |
| PHV908-2 | 0,7 | 0,9 | 0,6 | 0,4 | 0,0 |
| PHV908-3 | 0,5 | 0,7 | 0,8 | 0,5 | 0,2 |
| PHV908-4 | 0,5 | 0,4 | 0,4 | 0,5 | 0,2 |
| PHV908-5 | 0,8 | 0,5 | 0,4 | 0;3 | 0,3 |
| PHV908-6 | 1,2 | 0,4 | 0,3 | 0,2 | 0,0 |
| PHV908-7 | 1,7 | 0,4 | 0,2 | 0,0 | 0,1 |
| PHV908-8 | 2,2 | 0,5 | 0,3 | 0,2 | 0,1 |
| PHV908-9 | 2,0 | 0,6 | 0,3 | 0,3 | 0,1 |
| PHV908-10 | 2,2 | 0,9 | 0,5 | 0,3 | 0,2 |
| PHV908-11 | 1,7 | 1,0 | 0,5 | 0,3 | 0,0 |
| PHV908-12 | 1,3 | 0,8 | 0,4 | 0,7 | 0,2 |
| PHV908-13 | 1,3 | 1,0 | 0,5 | 0,3 | 0,1 |

**Tableau 2 :**

| **ID** | **E2** | **NS3** | **NS4B** | **NS5A** | **19D9D6** |
|---|---|---|---|---|---|
| PHV 910(M)-2 | 1,6 | 0,8 | 0,9 | 0,4 | 0,0 |
| PHV 910(M)-3 | 11,8 | 0,8 | 0,8 | 0,3 | 0,1 |
| PHV 910(M)-4 | 18,0 | 0,7 | 2,7 | 0,3 | 0,3 |
| PHV 910(M)-5 | 22,6 | 1,0 | 17,3 | 0,7 | 0.1 |

**Tableau 3 :**

| **ID** | **E2** | **NS3** | **NS4B** | **NS5A** | **19D9D6** |
|---|---|---|---|---|---|
| PHV911-2 | 0,2 | 0,5 | 0,7 | 0,2 | 0,1 |
| PHV911-3 | 0,2 | 0,6 | 0,7 | 0,3 | 0,3 |
| PHV911-4 | 0,8 | 9,5 | 2,0 | 0,5 | 0,1 |
| PHV911-5 | 1,7 | 17,1 | 3,7 | 0,4 | 0,1 |

**Tableau 4 :**

| **ID** | **E2** | **NS3** | **NS4B** | **NS5A** | **19D9D6** |
|---|---|---|---|---|---|
| PHV917(M)-1 | 1,0 | 1,0 | 0,7 | 0,7 | 0,2 |
| PHV917(M)-3 | 0,9 | 0,9 | 0,8 | 0,7 | 0,1 |
| PHV917(M)-4 | 0,6 | 0,6 | 1,3 | 1,3 | 0,2 |
| PHV917(M)-5 | 4,8 | 1,5 | 1,1 | 2,3 | 0,2 |
| PHV917(M)-6 | 1,9 | 3,7 | 0,9 | 1,4 | 0,0 |
| PHV917(M)-7 | 1,6 | 6,7 | 0,7 | 1,1 | 0,2 |
| PHV917(M)-8 | 1,5 | 7,0 | 0,7 | 1,1 | 0,7 |
| PHV917(M)-9 | 1,7 | 29,5 | 1,5 | 1,4 | 3,0 |
| PHV917(M)-10 | 1,6 | 32,8 | 0,8 | 1,1 | 1,2 |

**Tableau 5 :**

| **ID** | **E2** | **NS3** | **NS4B** | **NS5A** | **19D9D6** |
|---|---|---|---|---|---|
| 9044-1 | 0,4 | 2,4 | 0,6 | 0,4 | 0,5 |
| 9044-2 | 0,3 | 2,1 | 0,5 | 0,3 | 0,3 |
| 9044-3 | 0,3 | 2,0 | 0,5 | 0,3 | 0,3 |
| 9044-4 | 0,4 | 2,8 | 0,5 | 0,4 | 0,3 |
| 9044-5 | 0,8 | 6,7 | 0,5 | 2,0 | 0,4 |
| 9044-6 | 1,2 | 9,5 | 0,6 | 7,2 | 0,3 |

**Tableau 6 :**

| **ID** | **E2** | **NS3** | **NS4B** | **NS5A** | **19D9D6** |
|---|---|---|---|---|---|
| 6212-1 | 0,6 | 0,4 | 0,9 | 0,5 | 0,4 |
| 6212-2 | 0,8 | 0,4 | 1,0 | 0,5 | 0,1 |
| 6212-3 | 0,9 | 0,4 | 1,0 | 0,7 | 0,1 |
| 6212-4 | 0,5 | 0,5 | 0,4 | 0,3 | 0,2 |
| 6212-5 | 0,3 | 0,7 | 0,7 | 0,5 | 0,0 |
| 6212-6 | 0,3 | 1,4 | 0,8 | 0,5 | 0,2 |
| 6212-7 | 0,1 | 2,7 | 0,7 | 0,6 | 0,4 |
| 6212-8 | 0,3 | 15,0 | 0,9 | 0,6 | 0,3 |
| 6212-9 | 0,7 | 15,0 | 0,9 | 0,6 | 0,3 |

**Tableau 7 :**

| **ID** | **E2** | **NS3** | **NS4B** | **NS5A** | **19D9D6** |
|---|---|---|---|---|---|
| 6213-1 | 0,3 | 0,5 | 0,7 | 0,3 | 0,4 |
| 6213-2 | 0,3 | 0,5 | 0,5 | 0,4 | 0,3 |
| 6213-3 | 0,4 | 0,7 | 0,5 | 0,3 | 0,5 |
| 6213-4 | 0,3 | 0,6 | 0,5 | 0,4 | 0,3 |
| 6213-5 | 0,3 | 0,5 | 0,6 | 0,3 | 0,2 |
| 6213-6 | 0,3 | 0,5 | 0,4 | 0,3 | 0,7 |
| 6213-7 | 0,3 | 0,5 | 0,4 | 0,3 | 0,4 |
| 6213-8 | 0,2 | 0,3 | 0,4 | 0,2 | 0,6 |
| 6213-9 | 0,3 | 0,6 | 0,7 | 0,3 | 0,3 |
| 6213-10 | 0,4 | 1,0 | 0,6 | 0,5 | 0,3 |
| 6213-11 | 0,4 | 9,7 | 0,7 | 0,5 | 0,4 |
| 6213-12 | 0,4 | 10,6 | 0,7 | 0,4 | 0,3 |

**Tableau 8 :**

| **ID** | **E2** | **NS3** | **NS4B** | **NS5A** | **19D9D6** |
|---|---|---|---|---|---|
| 6214-1 | 1,0 | 0,9 | 1,1 | 0,8 | 0,5 |
| 6214-2 | 1,3 | 1,2 | 1,4 | 0,6 | 0,6 |
| 6214-3 | 0,8 | 0,7 | 0,9 | 0,5 | 0,3 |
| 6214-4 | 0,5 | 0,5 | 0,9 | 0,6 | 0,5 |
| 6214-5 | 0,5 | 0,4 | 0,8 | 0,6 | 0,3 |
| 6214-6 | 0,1 | 0,3 | 0,8 | 0,7 | 0,4 |
| 6214-7 | 0,3 | 0,4 | 0,9 | 0,8 | 0,5 |
| 6214-8 | 0,7 | 0,6 | 1,2 | 0,7 | 0,7 |
| 6214-9 | 1,3 | 1,0 | 1,3 | 0,9 | 0,4 |
| 6214-10 | 1,7 | 1,0 | 1,2 | 0,7 | 0,6 |
| 6214-11 | 1,5 | 1,3 | 0,9 | 0,6 | 0,3 |
| 6214-12 | 1,5 | 1,1 | 1,0 | 0,6 | 0,6 |
| 6214-13 | 1,5 | 1,2 | 1,1 | 0,7 | 0,3 |

**Tableau 9 :**

| **ID** | **E2** | **NS3** | **NS4B** | **NS5A** | **19D9D6** |
|---|---|---|---|---|---|
| 6215-1 | 0,6 | 3,3 | 0,6 | 0,1 | 0,3 |
| 6215-2 | 0,6 | 3,0 | 0,4 | 0,1 | 0,3 |
| 6215-3 | 1,6 | 3,2 | 0,8 | 0,2 | 0,2 |
| 6215-4 | 0,7 | 3,1 | 0,5 | 0,1 | 0,2 |

**Tableau 10 :**

| **ID** | **E2** | **NS3** | **NS4B** | **NS5A** | **19D9D6** |
|---|---|---|---|---|---|
| 6227-1 | 0,7 | 0,6 | 1,0 | 0,8 | 0,8 |
| 6227-2 | 0,4 | 0,4 | 0,9 | 0,7 | 0,7 |
| 6227-3 | 0,9 | 0,7 | 1,1 | 0,8 | 0,9 |
| 6227-4 | 1,2 | 0,9 | 1,2 | 0,9 | 0,8 |
| 6227-5 | 1,2 | 1,0 | 1,1 | 0,8 | 0,6 |
| 6227-6 | 10,6 | 1,3 | 1,4 | 0,8 | 0,5 |
| 6227-7 | 11,6 | 0,9 | 1,2 | 0,7 | 0,6 |

Ces tests sur les panels de séroconversion montrent que l'antigène E2 amène une détection plus précoce. Néanmoins l'effet précoce de détection de NS4 apparaît dans les tableaux 2, 3 4 et 8, celui de NS3 apparaît dans les tableaux 5, 6, 7 et 9 et celui de NS5 dans les tableaux 4 et 5.

### Exemple 3 : Essai avec les antigènes en mélange E2, NS3 1a, NS3 1b, NS4 et NS5. 3.1 Format de l'essai

### 1. sensibilisation des puits :

Les puits sont sensibilisés pendant 1 nuit à température ambiante sur microplaques MaxiSorb 96 puits (Nunc), avec 100 microlitres d'une solution d'antigènes suivante en mélange dilués dans le tampon PBS : E2 : 1microgramme/ml, NS3 génotype 1a : 0,5 microgramme/ml, NS3 génotype 1b : 0,5 microgramme/ml, NS4B : 1 microgramme/ml, NS5A : 1 microgramme/ml. Les plaques sont lavées avec 3 fois avec 300 microlitres de TBS-Tween 20 0,05% (tampon de lavage) puits passivées pendant 2 heures à 37°C dans un tampon TBS-Tween 20 0,05%, BSA 20g/l (Sigma), caséine 2,5g/l. Les plaques sont lavées 3 fois avec 300 microlitres en tampon de lavage.

### 2. Incubation de l'échantillon

Les puits sont incubés avec 100 microlitres d'échantillon dilués au 1/100 en tampon de passivation pendant 1 heure à 37°C puis lavés 3 fois avec 300 microlitres de tampon de lavage.

### 3. Incubation des conjugués

Les puits sont incubés pendant 1 heure à 37°C avec 100 microlitres d'une solution de anti-IgG humaines conjugué avec une enzyme Peroxydase de Raifort (HRP) (Jackson Immunoresearch) dilué à 0,1 microgramme/ml et anti-IgM humaines (bioMérieux) dilué 0,025 microgramme/ml en tampon de passivation. Les puits sont lavés 3 fois avec 300 microlitres de tampon de lavage.

### 4. Révélation

La réaction est révélée avec 100 microlitres d'un substrat O-phenyleneDiamine (Calbiochem) pendant 30 min à température ambiante et stoppée par ajout de 50 µl de H₂SO₄ 0,5M. Le signal est lu sur un lecteur de microplaques à 492 nm. La valeur seuil est calculée à partir du signal donné par la moyenne de 3 échantillons négatifs auquel est ajouté 3 fois l'écart type de ce signal. Le résultat peut être exprimé en ratio signal/seuil (s/co); un s/co supérieur à 1 est interprété comme positif.

### 3.2 Résultats sur les panels de séroconversion

La précocité de la détection en utilisant le format de test avec les antigènes en mélange est évaluée avec 11 panels de séroconversion fournis par Zeptometrix Corp ( panel HCV9044, 6212, 6213, 6214, 6215 et 6227) et Seracare/BBI diagnostics (panels PHV907, PHV908, PHV(910(M), PHV911 et PHV917(M)). Les échantillons sont des plasmas issus de prélèvements longitudinaux de personnes infectées par le VHC. Les résultats sont exprimés en Signal/Seuil; les valeurs supérieures à 1 sont considérées positives. La première colonne indique le code d'identification pour l'échantillon. La deuxième colonne le nombre de jours depuis le prélèvement initial pour un même patient (prélèvements longitudinaux). La troisième colonne indique le résultat exprimé en Signal/seuil obtenu avec un kit commercial Ortho EIA 3.0 qui est un kit commercial de détection précoce de la séroconversion pour le VHC qui utilise un peptide de Core (C22-3) et un polypeptide de NS3-NS4 (C200). La quatrième colonne indique le résultat exprimé en signal/seuil avec les antigènes en mélange. Chaque tableau correspond à une série de prélèvements pour un même patient.

**Tableau 11 :**

| **ID** | **Jours** | **Ortho EIA** **s/co** | **E2+NS3+NS4B+NS5A** **s/co** |
|---|---|---|---|
| PHV907-1 | 0 | 0 | 0,4 |
| PHV907-2 | 4 | 0 | 0,3 |
| PHV907-3 | 7 | 0 | 0,4 |
| PHV907-4 | 13 | 0.1 | 0,4 |
| PHV907-5 | 18 | 0.4 | 0,6 |
| PHV907-6 | 21 | 1 | 1,3 |
| PHV907-7 | 164 | 4.4 | 6,1 |

**Tableau 12 :**

| **ID** | **Jours** | **Ortho EIA** **s/co** | **E2+NS3+NS4B+NS5A** **s/co** |
|---|---|---|---|
| PHV908-01 | 0 | 0 | 0,3 |
| PHV908-02 | 3 | 0 | 0,5 |
| PHV908-03 | 5 | 0 | 0,5 |
| PHV908-04 | 11 | 0,1 | 0,8 |
| PHV908-05 | 13 | 0,3 | 0,9 |
| PHV908-06 | 19 | 1,7 | 1,3 |
| PHV908-07 | 25 | 4,9 | 1,8 |
| PHV908-08 | 27 | 4,9 | 1,7 |
| PHV908-09 | 32 | >5 | 2,0 |
| PHV908-10 | 35 | >5 | 2,5 |
| PHV908-11 | 41 | >5 | 2,4 |
| PHV908-12 | 45 | >5 | 2,9 |
| PHV908-13 | 48 | >5 | 2,6 |

**Tableau 13 :**

| **ID** | **Jours** | **Ortho EIA** s/co | **E2+NS3+NS4B+NS5A** s/co |
|---|---|---|---|
| 910-2 | 4 | 0 | 1,0 |
| 910-3 | 8 | 2,1 | 1,4 |
| 910-4 | 11 | >5 | 2,2 |
| 910-5 | 15 | >5 | 5,6 |

**Tableau 14 :**

| **ID** | **Jours** | **Ortho EIA** s/co | **E2+NS3+NS4B+NS5A** s/co |
|---|---|---|---|
| PHV911-02 | 3 | 0.0 | 0,5 |
| PHV911-03 | 14 | 1.6 | 0,4 |
| PHV911-04 | 21 | >5.0 | 3,3 |
| PHV911-05 | 24 | >5.0 | 5,2 |

**Tableau 15 :**

| ID | **Jours** | **Ortho EIA** s/co | **E2+NS3+NS4B+NS5A** s/co |
|---|---|---|---|
| 917-1 | 0 | 0 | 0,5 |
| 917-3 | 20 | 0 | 0,6 |
| 917-4 | 22 | 0 | 0,5 |
| 917-5 | 85 | >4,7 | 1,1 |
| 917-6 | 131 | >4,8 | 1,7 |
| 917-7 | 135 | >4,9 | 2,0 |
| 917-8 | 138 | >4,10 | 2,4 |
| 917-9 | 146 | >4,11 | 4,9 |
| 917-10 | 152 | >4,12 | 5,5 |

**Tableau 16 :**

| **ID** | **Jours** | **Ortho EIA** s/co | **E2+NS3+NS4B+NS5A** s/co |
|---|---|---|---|
| 9044-1 | 0 | 0.005 | 1,2 |
| 9044-2 | 4 | 0.003 | 0,6 |
| 9044-3 | 17 | 0.003 | 0,6 |
| 9044-4 | 21 | 0.124 | 0,8 |
| 9044-5 | 25 | 1.364 | 1,8 |
| 9044-6 | 29 | 1.864 | 3,3 |

**Tableau 17 :**

| **ID** | **Jours** | **Ortho EIA** s/co | **E2+NS3+NS4B+NS5A** s/co |
|---|---|---|---|
| 6212-1 | 0 | 0,003 | 0,2 |
| 6212-2 | 12 | 0,149 | 0,3 |
| 6212-3 | 14 | 0,297 | 0,2 |
| 6212-4 | 23 | 1,489 | 0,3 |
| 6212-5 | 26 | 1,866 | 0,4 |
| 6212-6 | 32 | 2,369 | 0,8 |
| 6212-7 | 37 | 2,461 | 1,0 |
| 6212-8 | 51 | 4,132 | 5,7 |

**Tableau 18 :**

| **ID** | **Jours** | **Ortho EIA** s/co | **E2+NS3+NS4B+NS5A** s/co |
|---|---|---|---|
| 6213-1 | 0 | 0,015 | 0,4 |
| 6213-2 | 2 | 0,012 | 0,2 |
| 6213-3 | 8 | 0,01 | 0,3 |
| 6213-4 | 11 | 0,009 | 0,3 |
| 6213-5 | 15 | 0,061 | 0,3 |
| 6213-6 | 17 | 0,009 | 0,2 |
| 6213-7 | 27 | 0,007 | 0,2 |
| 6213-8 | 29 | 0,009 | 0,7 |
| 6213-9 | 34 | 0,02 | 1,4 |
| 6213-10 | 36 | 0,51 | 1,4 |
| 6213-11 | 42 | 4,126 | 2,9 |
| 6213-12 | 46 | 4,126 | 3,3 |

**Tableau 19 :**

| ID | **Jours** | **Ortho EIA** s/co | **E2+NS3+NS4B+NS5A** s/co |
|---|---|---|---|
| 6215-1 | 0 | 0,01 | 0,4 |
| 6215-2 | 3 | 0,01 | 0,6 |
| 6215-3 | 10 | 0,01 | 0,8 |
| 6215-4 | 19 | 3,05 | 0,4 |

**Tableau 20 :**

| ID | **Jours** | **Ortho EIA** s/co | **E2+NS3+NS4B+NS5A** s/co |
|---|---|---|---|
| 6227-1 | 0 | 0,029 | 0,2 |
| 6227-2 | 21 | 0,067 | 0,2 |
| 6227-3 | 23 | 0,028 | 0,3 |
| 6227-4 | 41 | 0,042 | 0,2 |
| 6227-5 | 44 | 0,028 | 0,1 |
| 6227-6 | 74 | 3,686 | 0,9 |
| 6227-7 | 76 | 3,73 | 1,2 |

Ces tableaux comparatifs entre un kit commercial agréé FDA et marqué CE, contenant au moins un épitope de la protéine Core, et un prototype de recherche montrent que la combinaison d'antigènes choisis dans l'invention donnent des résultats très satisfaisants et beaucoup plus précoces. On observe toujours une variabilité inter patients. Par exemple le mélange d'antigènes selon l'exemple 3 permet une détection précoce de 8 jours dans le cas du tableau 18 mais moins précoce dans les tableaux 17, 19 ou 20. Il est donc nécessaire de rajouter la détection d'antigène pour augmenter la sensibilité.

### Exemple 4 : détection des anticorps dirigés contre les épitopes des protéines anti-E2, NS3, NS4B et NS5A comparée à détection de l'antigène Core

### 4.1 Format de l'essai

### 1. Format détection des anticorps anti-E2, NS3, NS4B, et NS5A

Le protocole est identique à celui décrit dans l'exemple 3.1 avec le même mélange d'antigènes fixés en fond de plaque

### 2. Format détection de l'antigène Core

Le protocole est identique à celui décrit dans l'exemple 2.1 paragraphe 1 a

### 4.2 Résultats sur 100 sérums HCV positifs tout venant

Les performances de l'essai ont été évaluées sur 100 échantillons de sérums issus de prélèvements de personnes infectés par le VHC.

Sur les 100 sérums testés, 2 sérums 57544 et 57302 présentent un résultat positif en détection de l'antigène Core et pas en détection des anticorps. Ces 2 sérums justifient donc le principe d'un test combo puisque ces 2 sérums, sans la détection de l'antigène, auraient donné un résultat négatif.

**Tableau 21 :**

| **ID serum** | **E2+NS3+NS4B+NS5A** s/co | **Ag Core** s/co |
|---|---|---|
| 57744 | 0,5 | 3,0 |
| 57302 | 0,4 | 3,0 |

### Exemple 5:Format de l'essai en mode combiné antigène et anticorps avec les antigènes E2, NS4B et NS5A en mélange avec l'anticorps 19D9D6.

### 5.1 Format de l'essai :

Le schéma du test est décrit dans la figure 1

### 1. Sensibilisation des puits :

Les puits sont sensibilisés pendant 2 heures à 37°C avec 100 microlitres d'une solution d'antigènes (E2, NS4B et NS5A) dilués chacun à 1 microgramme/ml en tampon TBS (Tris tamponné avec une solution saline), puis lavés avec 3x300 microlitres de TBS; et à nouveau fixés par adsorption passive pendant 2 heures à 37°C avec 200 microlitres d'une solution de l'anticorps anti-protéine Core 19D9D6 dilué à 4 microgrammes /ml dans le tampon TBS. Les plaques sont lavées avec 3 fois 300 microlitres de TBS-Tween 20 0,05% (tampon de lavage), puis passivées pendant une nuit à température ambiante dans un tampon TBS-Tween 20 0,05%, BSA 20g/l (Sigma), IgG souris 0,1g/l (Scantibodies laboratory) (tampon de passivation). Les plaques sont lavées avec 3 fois 300 microlitres en tampon de lavage.

### 2. Incubation de l'échantillon

Les puits sont incubés avec 200 microlitres d'échantillon dilués au 1/10 en tampon de passivation pendant 1 heure à 37°C puis lavés 5 fois avec 300 microlitres de tampon de lavage.

### 3. Incubation des conjugués

Les puits sont incubés pendant 1 heure à 37°C avec 200 microlitres d'une solution de l'anticorps anti-core 19D9D6 conjugué avec la Phosphatase alcaline dilué à 0,1 microgramme/ml et un Fab' anti-IgG humaines conjugué avec la Phosphatase alcaline dilué à 0,05 microgramme/ml en tampon de passivation. Les puits sont lavés 5 fois avec 300 microlitres de tampon de lavage.

### 4. Révélation

La réaction est révélée comme pour les exemples précédents.

### 5.2 Résultats

La précocité de la détection en utilisant le format de test avec les antigènes en mélange décrit dans l'exemple 2 est évaluée avec 2 panels de séroconversion fournis par Zeptometrix Corp (panel HCV6214) et Seracare BBI diagnostics (panel PHV(910(M)). Les échantillons sont des plasmas issus de prélèvements longitudinaux de personnes infectés par le VHC. Les résultats sont exprimés en Signal/Seuil; les valeurs supérieures à 1 sont considérées positives. La précocité de la détection est comparée par rapport à d'autres méthodes: dosage d'acides nucléiques (test Amplicor Roche PCR ou test Bayer bDNA 3.0) et Ortho EIA 3.0.

**Tableau 22 Panel de séroconversion PHV910(M) :**

| **ID** | **Jours** | **Roche PCR Amplicor** | **Ortho EIA 3** s/co | **Essai 5** s/co |
|---|---|---|---|---|
| 910-2 | 4 | >5E+5 | 0 | 1,8 |
| 910-3 | 8 | >5E+5 | 2,1 | 2,5 |
| 910-4 | 11 | >5E+5 | >5 | 3,6 |
| 910-5 | 15 | >5E+5 | >5 | 5,0 |

Les données montrent que le test combo détecte l'infection 4 jours plus tôt que le test commercial Ortho EIA 3. Le dosage des acides nucléiques indique la présence du virus dès le premier échantillon.

**Tableau 23 Panel de séroconversion 6214 :**

| **ID** | **jours** | **BAYER bDNA** Nombre de copies/ml | **Ortho EIA 3** s/co | **Essai 5** s/co |
|---|---|---|---|---|
| 6214-1 | 0 | 6 357 000 | 0,003 | 0,4 |
| 6214-2 | 2 | 6 998 000 | 0,002 | 0,3 |
| 6214-3 | 8 | 8 946 000 | 0,001 | 0,3 |
| 6214-4 | 10 | 6 910 000 | 0,003 | 0,3 |
| 6214-5 | 16 | 5 574 000 | 0,005 | 0,3 |
| 6214-6 | 18 | 3 312 000 | 0,003 | 0,3 |
| 6214-7 | 23 | 5 374 000 | 0,005 | 2,2 |
| 6214-8 | 27 | 11 200 000 | 0,012 | 3,5 |
| 6214-9 | 32 | 9 265 000 | 0,900 | 4,1 |
| 6214-10 | 34 | 6 278 000 | 2,643 | 3,9 |
| 6214-11 | 49 | 2 446 000 | 4,126 | 3,9 |
| 6214-12 | 53 | 2 939 000 | 4,126 | 4,0 |
| 6214-13 | 56 | 2 031 000 | 4,126 | 4,5 |

Les données montrent que le test combo de l'exemple 5 détecte l'infection 11 jours plus tôt que le test commercial Ortho EIA 3. Le dosage des acides nucléiques (méthode de référence) indique la présence du virus dès le premier échantillon.

### Exemple 6 : Format de l'essai en mode combiné antigène et anticorps avec les antigènes E2, NS3, NS4B et NS5A en mélange avec les anticorps 7G9B8 et 7G12A8.

### 6.1 Format de l'essai :

Le schéma du test est décrit dans la figure 1

### 1. Sensibilisation des puits

Les puits sont sensibilisés pendant 2 heures à 37°C avec 100 microlitres d'une solution d'antigènes dilué chacun à 1 microgramme/ml en tampon PBS ; puis lavées avec 3 fois 300 microlitres de PBS; et à nouveau sensibilisés pendant 2 heures à 37° avec 200 microlitres d'une solution des anticorps anti-Core 7G9B8 et 7G12A8 dilués à 0,5 microgramme/ml chacun dans le tampon PBS. Les plaques sont lavées avec 3 fois 300 microlitres de PBS-Tween 20 0,05% (tampon de lavage) puits passivées pendant une nuit à température ambiante dans un tampon PBS-Tween 20 0,05%, caséine 1% (tampon de passivation). Les plaques sont lavées avec 3 fois 300 microlitres en tampon de lavage.

### 2. Incubation de l'échantillon

Les puits sont incubés avec 200 microlitres d'échantillon dilués au 1/3 en tampon TBS-Tween 0,05%, sérum de chèvre 10% pendant 1 heure à 37°C puis lavés 3 fois avec 300 microlitres de tampon de lavage.

### 3. Incubation des conjugués

Les puits sont incubés pendant 1 heure à 37°C avec 200 microlitres d'une solution d'anticorps anti-core 19D9D6 conjugués avec la Horseradish-Peroxydase (HRP) dilués à 1 microgramme/ml, anti-IgG humaines conjuguées (HRP) diluées à 0,1 microgramme/ml, anti-IgM humaines conjuguées (HRP) diluées à 0,1 microgramme/ml en tampon de passivation. Les puits sont lavés 3 fois avec 300 microlitres de tampon de lavage.

### 4. Révélation

La réaction est révélée avec 100 microlitres d'un substrat O-phenyleneDiamine (Calbiochem) pendant 30 min à température ambiante et stoppée par ajout de 50 microlitres de H₂SO₄ 0,5M. Le signal est lu sur un lecteur de microplaques à 492nm.

### 6.2 Résultats sur les panels de séroconversion

La précocité de la détection est évaluée avec 11 panels de séroconversion fournis par Zeptometrix Corp (panel HCV9044, 6212, 6213, 6214, 6215 et 6227) et Seracare BBI diagnostics (panels PHV 907, PHV908, PHV910(M), PHV911 et PHV917(M)). Les échantillons sont des plasmas issus de prélèvements longitudinaux de personnes infectés par le VHC. La valeur seuil est calculée à partir du signal donné par la moyenne de 3 échantillons négatifs additionné de 3 fois l'écart-type de se signal. Le résultat peut être exprimé en ratio signal/seuil (s/co); un s/co supérieur à 1 est interprété comme positif.

**Tableau 24 :**

| **ID** | **jours** | **Ortho EIA** s/co | **Essai 6** |
|---|---|---|---|
| PHV907-1 | 0 | 0 | 1,4 |
| PHV907-2 | 4 | 0 | 1,3 |
| PHV907-3 | 7 | 0 | 1,2 |
| PHV907-4 | 13 | 0.1 | 1,4 |
| PHV907-5 | 18 | 0.4 | 1,4 |
| PHV907-6 | 21 | 1 | 1,6 |
| PHV907-7 | 164 | 4.4 | 1,9 |

**Tableau 25 :**

| **ID** | **jours** | **Ortho EIA** s/co | **Essai 6** |
|---|---|---|---|
| PHV908-01 | 0 | 0 | 1,1 |
| PHV908-02 | 3 | 0 | 0,9 |
| PHV908-03 | 5 | 0 | 1,1 |
| PHV908-04 | 11 | 0,1 | 1,1 |
| PHV908-05 | 13 | 0,3 | 1,4 |
| PHV908-06 | 19 | 1,7 | 1,5 |
| PHV908-07 | 25 | 4,9 | 1,6 |
| PHV908-08 | 27 | 4,9 | 1,7 |
| PHV908-09 | 32 | >5 | 1,3 |
| PHV908-10 | 35 | >5 | 1,5 |
| PHV908-11 | 41 | >5 | 1,4 |
| PHV908-12 | 45 | >5 | 1,6 |

**Tableau 26 :**

| **ID** | **jours** | **Ortho EIA** s/co | **Essai 6** |
|---|---|---|---|
| 910-2 | 4 | 0 | 1,5 |
| 910-3 | 8 | 2,1 | 1,6 |
| 910-4 | 11 | >5 | 1,9 |
| 910-5 | 15 | >5 | 1,8 |

**Tableau 27 :**

| **ID** | **jours** | **Ortho EIA** s/co | **Essai 6** |
|---|---|---|---|
| PHV911-02 | 3 | 0.0 | 0,8 |
| PHV911-03 | 14 | 1.6 | 1,0 |
| PHV911-04 | 21 | >5.0 | 1,7 |
| PHV911-05 | 24 | >5.0 | 1,7 |

**Tableau 28 :**

| **ID** | **jours** | **Ortho EIA** s/co | **Essai 6** |
|---|---|---|---|
| 917-1 | 0 | 0 | 1,4 |
| 917-3 | 20 | 0 | 1,0 |
| 917-4 | 22 | 0 | 1,2 |
| 917-5 | 85 | >4,7 | 1,2 |
| 917-6 | 131 | >4,8 | 1,4 |
| 917-7 | 135 | >4,9 | 1,4 |
| 917-8 | 138 | >4,10 | 1,5 |
| 917-9 | 146 | >4,11 | 1,7 |

**Tableau 29 :**

| **ID** | **jours** | **Ortho EIA** s/co | **Essai 6** |
|---|---|---|---|
| 9044-1 | 0 | 0.005 | 1,1 |
| 9044-2 | 4 | 0.003 | 1,1 |
| 9044-3 | 17 | 0.003 | 1,3 |
| 9044-4 | 21 | 0.124 | 1,1 |
| 9044-5 | 25 | 1.364 | 1,3 |
| 9044-6 | 29 | 1.864 | 1,4 |

**Tableau 30 :**

| **ID** | **jours** | **Ortho EIA** s/co | **Essai 6** |
|---|---|---|---|
| 6212-1 | 0 | 0,003 | 0,8 |
| 6212-2 | 12 | 0,149 | 1,0 |
| 6212-3 | 14 | 0,297 | 0,8 |
| 6212-4 | 23 | 1,489 | 1,1 |
| 6212-5 | 26 | 1,866 | 1,0 |
| 6212-6 | 32 | 2,369 | 1,1 |
| 6212-7 | 37 | 2,461 | 1,3 |
| 6212-8 | 51 | 4,132 | 1,6 |
| 6212-9 | 53 | 4,132 | 1,4 |

**Tableau 31 :**

| **ID** | **Jours** | **Ortho EIA** s/co | **Essai 6** |
|---|---|---|---|
| 6213-1 | 0 | 0,015 | 1,3 |
| 6213-2 | 2 | 0,012 | 1,3 |
| 6213-3 | 8 | 0,01 | 1,5 |
| 6213-4 | 11 | 0,009 | 1,3 |
| 6213-5 | 15 | 0,061 | 1,1 |
| 6213-6 | 17 | 0,009 | 1,4 |
| 6213-7 | 27 | 0,007 | 1,2 |
| 6213-8 | 29 | 0,009 | 1,3 |
| 6213-9 | 34 | 0,02 | 1,3 |
| 6213-10 | 36 | 0,51 | 1,2 |
| 6213-11 | 42 | 4,126 | 1,7 |
| 6213-12 | 46 | 4,126 | 1,5 |

**Tableau 32 :**

| **ID** | **jours** | **Ortho EIA** s/co | **Essai 6** |
|---|---|---|---|
| 6214-1 | 0 | 0,003 | 1,1 |
| 6214-2 | 2 | 0,002 | 1,2 |
| 6214-3 | 8 | 0 | 1,0 |
| 6214-4 | 10 | 0,003 | 1,2 |
| 6214-5 | 16 | 0,005 | 1,0 |
| 6214-6 | 18 | 0,003 | 1,0 |
| 6214-7 | 23 | 0,005 | 1,1 |
| 6214-8 | 27 | 0,012 | 1,1 |
| 6214-9 | 32 | 0,9 | 1,3 |
| 6214-10 | 34 | 2,643 | 1,2 |
| 6214-11 | 49 | 4,126 | 1,7 |
| 6214-12 | 53 | 4,126 | 1,8 |

**Tableau 33 :**

| **ID** | **jours** | **Ortho EIA** s/co | **Essai 6** |
|---|---|---|---|
| 6215-1 | 0 | 0,01 | 0,7 |
| 6215-2 | 3 | 0,01 | 0,7 |
| 6215-3 | 10 | 0,01 | 1,2 |
| 6215-4 | 19 | 3,05 | 0,9 |

**Tableau 34 :**

| **ID** | **Jours** | **Ortho EIA** s/co | **Essai 6** |
|---|---|---|---|
| 6227-1 | 0 | 0,029 | 0,7 |
| 6227-2 | 21 | 0,067 | 0,6 |
| 6227-3 | 23 | 0,028 | 0,6 |
| 6227-4 | 41 | 0,042 | 1,1 |
| 6227-5 | 44 | 0,028 | 1,0 |
| 6227-6 | 74 | 3,686 | 1,3 |
| 6227-7 | 76 | 3,73 | 1,5 |

La combinaison utilisée dans l'essai 6 donne de meilleurs résultats que le test Ortho pour la plupart des panels et détecte avec au moins la même sensibilité pour les tableaux 27 et 30.

### Bibliographie

Alter M.J. et al, N. Engl. J. Med., 1992, 327, 1899-1905.
Arribillaga L. et al , Vaccine , 2002, 21, 202-210.
Cheynet V. et al, Prot. Expr. Purif., 1993, 4(5), 367-472.
Choo Q.L.et al, Science, 1989, 244, 359-362.
Choo Q.L.et al, Proc. Nat. Acad. Sci. USA, 1991, 88, 2451-2455.
Courouce AM et al, Lancet, 1994, 343, 853-854.
Garson JA et al, Lancet. 1990, 336, 1022-1025.
Gretch D.R. et al, Anal. Biochem, 1987, 163, 270-277.
Han J.H. et al, Proc. Nat. Acad. Sci. USA, 1991, 88(5), 1711-1715.
Jolivet-Reynaud C. et al, Journal of Medical Virology, 1998, 56, 300-309.
Kato N. et al, Virus Res., 1992, 22, 107-123.
Köhler G. et Milstein C., Nature, 1975, 256, 45-47.
Köhler G. et Milstein C., Eur. J. Immunology, 1976, 6, 511-519.
Ménez A. et al, J. Immunology, 2003, 170, 1917-1924.
Mimms L. et al, Lancet, 1990, 336, 1590-1591.
Takahashi K. et al, J. Gen. Virol., 1992, 73, 667-672.

## Revendications

1. Support solide de test immunologique pour la détection du VHC sur lequel sont fixés :
a) au moins un anticorps dirigé contre la protéine de Core du VHC et
b) un polypeptide consistant en (i) un peptide de la protéine E2 du VHC choisi parmi la protéine E2 elle-même et un ou plusieurs de ses épitopes et (ii) un peptide des protéines E1, NS4B et/ou NS5A du VHC choisi parmi les protéines elles-mêmes et un ou plusieurs de leurs épitopes, et, le cas échéant, en (iii) un peptide de la protéine NS3 choisi parmi la protéine elle-même et un ou plusieurs de ses épitopes,
étant entendu qu'aucun polypeptide comprenant un épitope de la protéine Core du VHC n'est fixé sur le support solide.

2. Support solide de test immunologique pour la détection du VHC selon la revendication 1, **caractérisé en ce que** le peptide (ii) est choisi parmi les protéines NS4B, NS5A et un ou plusieurs de leurs épitopes.

3. Support solide de test immunologique pour la détection du VHC selon la revendication 2, **caractérisé en ce que** le peptide (ii) comprend au moins 10 acides aminés contiguës de la séquence SEQ ID n°5 et/ou la SEQ ID n°7.

4. Support solide de test immunologique pour la détection du VHC selon la revendication 1 à 3, **caractérisé en ce que** le peptide (i) comprend au moins 10 acides aminés contiguës de la séquence SEQ ID n° 2.

5. Support solide de test immunologique pour la détection du VHC selon la revendication 1 à 4, **caractérisé en ce que** le polypeptide b) ne comprend pas de peptide (iii).

6. Support solide de test immunologique pour la détection du VHC sur lequel sont fixés :
a) au moins un anticorps dirigé contre la protéine de Core du VHC,
b) un peptide de la protéine E2 du VHC choisi parmi la protéine E2 elle-même ou un ou plusieurs de ses épitopes et
c) un peptide des protéines E1, NS4B et/ou NS5A du VHC choisi parmi les protéines elles-mêmes et un ou plusieurs de leurs épitopes, avec, le cas échéant
d) un peptide de la protéine NS3 choisi parmi la protéine elle-même et un ou plusieurs de ses épitopes,
étant entendu qu'aucun polypeptide comprenant un épitope de la protéine Core du VHC n'est fixé sur le support solide.

7. Support solide de test immunologique pour la détection du VHC selon la revendication 6, **caractérisé en ce que** le peptide c) est choisi parmi les protéines NS4B, NS5A et un ou plusieurs de leurs épitopes.

8. Support solide de test immunologique pour la détection du VHC selon la revendication 7, **caractérisé en ce que** le peptide c) comprend au moins 10 acides aminés contiguës de la séquence SEQ ID n°5 et/ou la SEQ ID n°7.

9. Support solide de test immunologique pour la détection du VHC selon la revendication 6 à 8, **caractérisé en ce que** le peptide b) comprend au moins 10 acides aminés contiguës de la séquence SEQ ID n° 2.

10. Support solide de test immunologique pour la détection du VHC selon la revendication 6 à 9, lequel ne comprend pas de peptide de la protéine NS3 d).

11. Procédé de détection *in vitro* d'une infection par le VHC dans un échantillon biologique comprenant la détection d'au moins un antigène du VHC et d'un anticorps dirigé contre le VHC présent dans l'échantillon biologique et dans lequel
on dispose d'un support selon l'une quelconque des revendications 1 à 10,
on incube ledit support avec l'échantillon biologique dans des conditions permettant le formation de complexes antigènes-anticorps,
on révèle les complexes antigènes-anticorps formés.

## Patentansprüche

1. Fester Träger für einen immunologischen Test zum Nachweisen von HCV, auf dem fixiert sind:
a) mindestens ein Antikörper gegen das HCV-Core-Protein und
b) ein Polypeptid bestehend aus (i) einem Peptid des HCV-E2-Proteins, ausgewählt aus dem E2-Protein selbst und einem oder mehreren seiner Epitope, und (ii) einem Peptid der HCV-Proteine E1, NS4B und/oder NS5A, ausgewählt aus den Proteinen selbst und einem oder mehreren ihrer Epitope, sowie gegebenenfalls (iii) einem Peptid des NS3-Proteins, ausgewählt aus dem Protein selbst und einem oder mehreren seiner Epitope,
mit der Maßgabe, dass kein Polypeptid, das ein Epitop des HCV-Core-Proteins umfasst, auf dem festen Träger fixiert ist.

2. Fester Träger für einen immunologischen Test zum Nachweisen von HCV nach Anspruch 1, **dadurch gekennzeichnet, dass** das Peptid (ii) aus den Proteinen NS4B, NS5A und einem oder mehreren ihrer Epitope ausgewählt ist.

3. Fester Träger für einen immunologischen Test zum Nachweisen von HCV nach Anspruch 2, **dadurch gekennzeichnet, dass** das Peptid (ii) mindestens 10 aufeinanderfolgende Aminosäuren der Sequenz SEQ ID Nr. 5 und/oder SEQ ID Nr. 7 umfasst.

4. Fester Träger für einen immunologischen Test zum Nachweisen von HCV nach Anspruch 1 bis 3, **dadurch gekennzeichnet, dass** das Peptid (i) mindestens 10 aufeinanderfolgende Aminosäuren der Sequenz SEQ ID Nr. 2 umfasst.

5. Fester Träger für einen immunologischen Test zum Nachweisen von HCV nach Anspruch 1 bis 4, **dadurch gekennzeichnet, dass** das Polypeptid b) kein Peptid (iii) umfasst.

6. Fester Träger für einen immunologischen Test zum Nachweisen von HCV, auf dem fixiert sind:
a) mindestens ein Antikörper gegen das HCV-Core-Protein und
b) ein Peptid des HCV-Proteins E2, ausgewählt aus dem Protein E2 selbst und einem oder mehreren seiner Epitope, und
c) ein Peptid der HCV-Proteine E1, NS4B und/oder NS5A, ausgewählt aus den Proteinen selbst und einem oder mehreren ihrer Epitope, sowie gegebenenfalls
d) ein Peptid des Proteins NS3, ausgewählt aus dem Protein selbst und einem oder mehreren seiner Epitope,
mit der Maßgabe, dass kein Polypeptid, das ein Epitop des HCV-Core-Proteins umfasst, auf dem festen Träger fixiert ist.

7. Fester Träger für einen immunologischen Test zum Nachweisen von HCV nach Anspruch 6, **dadurch gekennzeichnet, dass** das Peptid c) aus den Proteinen NS4B, NS5A und einem oder mehreren ihrer Epitope ausgewählt ist.

8. Fester Träger für einen immunologischen Test zum Nachweisen von HCV nach Anspruch 7, **dadurch gekennzeichnet, dass** das Peptid c) mindestens 10 aufeinanderfolgende Aminosäuren der Sequenz SEQ ID Nr. 5 und/oder SEQ ID Nr. 7 umfasst.

9. Fester Träger für einen immunologischen Test zum Nachweisen von HCV nach Anspruch 6 bis 8, **dadurch gekennzeichnet, dass** das Peptid b) mindestens 10 aufeinanderfolgende Aminosäuren der Sequenz SEQ ID Nr. 2 umfasst.

10. Fester Träger für einen immunologischen Test zum Nachweisen von HCV nach Anspruch 6 bis 9, der kein Peptid des Proteins NS3 d) umfasst.

11. Verfahren für den in-vitro-Nachweis einer HCV-Infektion in einer biologischen Probe, umfassend das Nachweisen von mindestens einem HCV-Antigen und von einem gegen HCV gerichteten Antikörper in der biologischen Probe, wobei man
über einen Träger nach einem der Ansprüche 1 bis 10 verfügt,
den Träger mit der biologischen Probe unter Bedingungen, die die Bildung von Antigen-Antikörper-Komplexen gestatten, inkubiert,
die gebildeten Antigen-Antikörper-Komplexe sichtbar macht.

## Claims

1. A solid support for an immunological test for detecting HCV, on which the following are attached:
a) at least one antibody directed against the HCV Core protein, and
b) a polypeptide consisting of (i) a peptide of the E2 protein of HCV, chosen from the E2 protein itself and one or more of its epitopes, and (ii) a peptide of the E1, NS4B and/or NS5A proteins of HCV, chosen from the proteins themselves and one or more of their epitopes, and, where appropriate, (iii) a peptide of the NS3 protein, chosen from the protein it-self and one or more of its epitopes,
it being understood that no polypeptide comprising an epitope of the HCV Core protein is attached to the solid support.

2. The solid support for an immunological test for detecting HCV as claimed in claim 1, **characterized in that** the peptide (ii) is chosen from the NS4B and NS5A proteins and one or more of their epitopes.

3. The solid support for an immunological test for detecting HCV as claimed in claim 2, **characterized in that** the peptide (ii) comprises at least 10 contiguous amino acids of the sequence SEQ ID No. 5 and/or SEQ ID No. 7.

4. The solid support for an immunological test for detecting HCV as claimed in claims 1 to 3, **characterized in that** the peptide (i) comprises at least 10 contiguous amino acids of the sequence SEQ ID No. 2.

5. The solid support for an immunological test for detecting HCV as claimed in claims 1 to 4, **characterized in that** the polypeptide b) does not comprise any peptide (iii).

6. A solid support for an immunological test for detecting HCV, on which the following are attached:
a) at least one antibody directed against the HCV Core protein,
b) a peptide of the E2 protein of HCV, chosen from the E2 protein itself or one or more of its epitopes, and
c) a peptide of the E1, NS4B and/or NS5A proteins of HCV, chosen from the proteins themselves and one or more of their epitopes, with, where appropriate,
c) a peptide of the NS3 protein, chosen from the protein itself and one or more its epitopes,
it being understood that no polypeptide comprising an epitope of the HCV Core protein is attached to the solid support.

7. The solid support for an immunological test for detecting HCV as claimed in claim 6, **characterized in that** the peptide c) is chosen from the NS4B and NS5A proteins and one or more of their epitopes.

8. The solid support for an immunological test for detecting HCV as claimed in claim 7, **characterized in that** the peptide c) comprises at least 10 contiguous amino acids of the sequence SEQ ID No. 5 and/or SEQ ID No. 7.

9. The solid support for an immunological test for detecting HCV as claimed in claims 6 to 8, **characterized in that** the peptide b) comprises at least 10 contiguous amino acids of the sequence SEQ ID No. 2.

10. The solid support for an immunological test for detecting HCV as claimed in claims 6 to 9, which does not comprise any peptide of the NS3 protein d).

11. A method for detecting, *in vitro,* an HCV infection in a biological sample, which comprises detecting at least one HCV antigen and an antibody directed against HCV present in the biological sample, and in which:
a support as claimed in any one of claims 1 to 10 is provided,
said support is incubated with the biological sample under conditions which allow the formation of antigen-antibody complexes,
the antigen-antibody complexes formed are revealed.
